(19) Europäisches Patentamt
European Patent Office
Office européen des brevets

(11) EP 3 934 608 B1

(12) EUROPÄISCHE PATENTSCHRIFT

(45) Veröffentlichungstag und Bekanntmachung des Hinweises auf die Patenterteilung:
**29.03.2023 Patentblatt 2023/13**

(21) Anmeldenummer: **20702754.1**

(22) Anmeldetag: **24.01.2020**

(51) Internationale Patentklassifikation (IPC):
***A61K 8/34*** *(2006.01)* ***A61K 8/898*** *(2006.01)*
***A61Q 5/00*** *(2006.01)* ***A61Q 5/06*** *(2006.01)*
***A61Q 5/08*** *(2006.01)* ***A61Q 5/10*** *(2006.01)*

(52) Gemeinsame Patentklassifikation (CPC):
**A61K 8/342; A61K 8/898; A61Q 5/065; A61Q 5/08; A61Q 5/10;** A61K 2800/30; A61K 2800/805

(86) Internationale Anmeldenummer:
**PCT/EP2020/051823**

(87) Internationale Veröffentlichungsnummer:
**WO 2020/177946 (10.09.2020 Gazette 2020/37)**

# (54) VERFAHREN ZUR HERSTELLUNG UND ANWENDUNG VON HAARBEHANDLUNGSMITTELN MIT ORGANISCHEN C1-C6-ALKOXY-SILANEN

METHOD FOR PREPARING AND USING HAIR TREATMENT AGENTS WITH ORGANIC C1-C6-ALKOXY-SILANES

PROCÉDÉ POUR PRODUIRE ET UTILISER DES AGENTS DE TRAITEMENT CAPILLAIRE COMPORTANT DES ALCOXYSILANES ORGANIQUES EN C1-C6

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.03.2019 DE 102019203081**

(43) Veröffentlichungstag der Anmeldung:
**12.01.2022 Patentblatt 2022/02**

(73) Patentinhaber: **Henkel AG & Co. KGaA**
**40589 Düsseldorf (DE)**

(72) Erfinder:
- **LECHNER, Torsten**
  **40764 Langenfeld (DE)**
- **LOHR, Christoph**
  **40822 Mettmann (DE)**
- **WALTER, Andreas**
  **40880 Ratingen (DE)**
- **THIESSIES, Claus-Peter**
  **40593 Düsseldorf (DE)**
- **SCHOEPGENS, Juergen**
  **41366 Schwalmtal (DE)**

(56) Entgegenhaltungen:
**EP-B1- 2 168 633 WO-A1-2020/035186**

- **ARKLES B ET AL: "Factors contributing to the stability of alkoxysilanes in aqueous solution", 1. Januar 1992 (1992-01-01), SILANES AND OTHER COUPLING AGENTS, VSP, UTRECHT, NL, PAGE(S) 91 - 104, XP007918498, ISBN: 978-90-6764-142-5**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die vorliegende Anmeldung liegt auf dem Gebiet der Kosmetik und betrifft ein Verfahren zur Herstellung von Haarbehandlungsmitteln. Im Rahmen des erfindungsgemäßen Verfahrens werden ein oder mehrere organische $C_1$-$C_6$-Alkoxy-Silane mit Wasser zur Reaktion gebracht, und die bei dieser Reaktion freigesetzten $C_1$-$C_6$-Alkohole werden möglichst vollständig aus dem Reaktionsgemisch entfernt. Als weitere Schritte umfasst das erfindungsgemäße Verfahren optional die Zugabe eines oder mehrerer kosmetischer Inhaltsstoffe zu dieser Zubereitung und die Abfüllung der Zubereitung in eine Verpackungseinheit.

Ein zweiter Gegenstand ist ein Mittel zur Behandlung von keratinischem Material, umfassend eine Zubereitung in einer Verpackungseinheit, die nach vorgenannten Verfahren hergestellt wurde.

Ein dritter Gegenstand der vorliegenden Erfindung ist eine Mehrkomponenten-Verpackungseinheit (Kit-of-parts) zum Färben von keratinischem Material, welche getrennt konfektioniert in zwei Verpackungseinheiten die kosmetischen Zubereitungen (A) und (B) umfasst, wobei es sich bei der Zubereitung (A) um eine Zubereitung des ersten Erfindungsgegenstandes handelt und die Zubereitung (B) mindestens eine farbgebende Verbindung enthält.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Schamponierungen besonders widerstandsfähig sind.

Bei den in der EP 2168633 B1 eingesetzten organischen Silicium-Verbindungen handelt es sich um reaktive Verbindungen aus der Klasse der Alkoxy-Silane. Diese Alkoxy-Silane hydrolysieren in Gegenwart von Wasser mit hoher Geschwindigkeit und bilden - abhängig von den jeweils eingesetzten Mengen an Alkoxy-Silan und Wasser - Hydrolyseprodukte und/oder Kondensationsprodukte aus. Der Einfluss der in dieser Reaktion eingesetzten Wassermenge auf die Eigenschaften des Hydrolyse- bzw. Kondensationsproduktes werden beispielsweise in WO 2013068979 A2 beschrieben.

Wenn diese Hydrolyse- bzw. Kondensationsprodukte auf keratinischem Material angewendet werden, bildet sich auf dem Keratinmaterial ein Film oder auch ein Coating aus, welches das Keratinmaterial vollständig umhüllt und auf diese Weise die Eigenschaften des Keratinmaterials stark beeinflusst. Mögliche Anwendungsbereiche sind beispielsweise das permanente Styling oder auch die permanente Formveränderung von Keratinfasern. Hierbei werden die Keratinfasern mechanisch in die gewünschte Form gebracht und in dieser Form dann durch Ausbildung des vorbeschriebenen Coatings fixiert. Eine weitere ganz besonders gut geeignete Anwendungsmöglichkeit ist die Färbung von Keratinmaterial; im Rahmen dieser Anwendung wird das Coating bzw. der Film in Gegenwart einer farbgebenden Verbindung, zum Beispiel eines Pigments, erzeugt. Der durch das Pigment gefärbte Film verbleibt auf dem Keratinmaterial bzw. den Keratinfasern und resultiert in überraschend waschbeständigen Färbungen.

Der große Vorteil des auf Alkoxy-Silanen basierenden Färbeprinzips liegt darin, dass die hohe Reaktivität dieser Verbindungsklasse ein sehr schnelles Coating ermöglicht. So können bereits nach sehr kurzen Anwendungszeiträumen von nur wenigen Minuten extrem gute Färbeergebnisse erzielt werden. Neben diesen Vorteilen birgt die hohe Reaktivität

der Alkoxy-Silane jedoch auch einige Nachteile. So können bereits geringfügige Änderungen der Produktions- und Anwendungsbedingungen, wie beispielsweise die Änderung von Luftfeuchtigkeit und/oder Temperatur, zu starken Schwankungen der Produktleistung führen. Vor allem haben die zu dieser Erfindung führenden Arbeiten gezeigt, dass die Alkoxy-Silane extrem sensibel auf die Bedingungen reagieren, die bei der Herstellung der Keratinbehandlungsmittel herrschen.

Analytische Untersuchungen haben gezeigt, dass bei der Herstellung verschiedener Silan-Gemische und Blends komplexe Hydrolyse- und Kondensations-Reaktionen ablaufen, die abhängig von den gewählten Reaktionsbedingungen zu oligomeren Produkten unterschiedlicher Molekülgröße führen. In diesem Zusammenhang hat sich herausgestellt, dass das Molekulargewicht dieser Silan-Oligomere großen Einfluss auf die späteren Produkteigenschaften nehmen kann. Werden bei der Herstellung die falschen Bedingungen gewählt, so kann dies zur Ausbildung von zu großen oder zu kleinen Silan-Kondensaten führen, wodurch die spätere Produktleistung, insbesondere das spätere Färbevermögen auf dem Keratinmaterial, negativ beeinflusst wird.

Es war die Aufgabe der vorliegenden Anmeldung, ein optimiertes Verfahren für die Herstellung von Keratinbehandlungsmitteln aufzufinden. Die in diesem Verfahren eingesetzten Alkoxy-Silane sollten auf gezielte Weise so hergestellt werden, dass bei einer anschließenden Anwendung die optimalen anwendungstechnischen Eigenschaften erzielt werden konnten. Insbesondere sollten die auf diesem Wege hergestellten Mittel eine verbesserte Färbeleistung besitzen, d.h. bei ihrer Anwendung in einem Färbeverfahren sollten Färbungen mit höherer Farbintensität und verbesserten Echtheitseigenschaften, insbesondere mit einer verbesserten Waschechtheit und einer verbesserten Reibechtheit, erzielt werden.

Überraschenderweise hat sich nun herausgestellt, dass die vorgenannte Aufgabe hervorragend gelöst werden kann, wenn ein Herstellungsverfahren gewählt wird, bei dem eine gezielte Hydrolyse von organischen $C_1$-$C_6$-Alkoxy-Silanen vorgenommen, und die der bei dieser Reaktion freigesetzten Alkohole möglichst vollständig entfernt werden. Die auf diese Weise hergestellten Zubereitungen können gegebenenfalls mit weiteren kosmetischen Inhaltsstoffen versetzt werden. Dann erfolgt die Abfüllung in eine Verpackungseinheit.

Ein erster Gegenstand der vorliegenden Erfindung ist ein Verfahren zur Herstellung eines Mittels zur Behandlung von keratinischem Material, insbesondere menschlichen Haaren, umfassend die folgenden Schritte:

(1) Mischen eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,

dadurch gekennzeichnet, dass der Gesamtgehalt an $C_1$-$C_6$-Alkoholen in der nach Schritt (4) erhaltenen Zubereitung - bezogen auf das Gesamtgewicht der Zubereitung - unterhalb von 10,0 Gew.-% liegt.

Es hat sich gezeigt, dass Haarbehandlungsmittel, die durch dieses erfindungsgemäße Verfahren hergestellt wurden, bei Einsatz in einem Färbeprozess zu sehr intensiven und gleichmäßigen Färbungen mit sehr guter Deckkraft, Reibechtheit und Waschechtheit führten.

Mittel zur Behandlung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter Mitteln zur Behandlung von keratinischem Material werden beispielsweise Mittel zur Färbung des Keratinmaterials, Mittel zur Umformung oder Formgebung von keratinischem Material, insbesondere keratinischen Fasern, oder auch Mittel zur Konditionierung bzw. zur Pflege des keratinischen Materials verstanden. Besonders gute Eignung zeige die über das erfindungsgemäße Verfahren hergestellten Mittel zur Färbung von keratinischem Material, insbesondere zur Färbung von keratinischen Fasern, bei denen es sich besonders bevorzugt um menschliche Haare handelt.

Der Begriff "Mittel zur Färbung" wird im Rahmen dieser Erfindung für eine durch Einsatz von farbgebenden Verbindungen, wie beispielsweise thermochromen und photochromen Farbstoffen, Pigmenten, Mica, direktziehenden Farbstoffen und/oder Oxidationsfarbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich *in situ* durch Oligomerisierung bzw. Kondensation des oder der organischen Siliciumverbindungen, wobei die farbgebende(n) Verbindung(en) mit diesem Film bzw. diesem Coating wechselwirken oder in diesen eingelagert

werden.

Mischen von $C_1$-$C_6$-Alkoxv-Silan(en) mit Wasser

Bei Schritt (1) des erfindungsgemäßen Verfahrens handelt es sich um die Reaktion oder auch Umsetzung von einem oder mehreren organischen $C_1$-$C_6$-Alkoxy-Silanen mit Wasser. Zur Initiierung dieser Reaktion werden das bzw. die $C_1$-$C_6$-Alkoxy-Silane mit Wasser gemischt.

Mit anderen Worten ist der erste Gegenstand der vorliegenden Erfindung ist ein Verfahren zur Herstellung eines Mittels zur Behandlung von keratinischem Material, insbesondere menschlichen Haaren, umfassend die folgenden Schritte:

(1) Reaktion eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,

dadurch gekennzeichnet, dass der Gesamtgehalt an $C_1$-$C_6$-Alkoholen in der nach Schritt (4) erhaltenen Zubereitung - bezogen auf das Gesamtgewicht der Zubereitung - unterhalb von 10,0 Gew.-% liegt.

Bei dem oder den organischen $C_1$-$C_6$-Alkoxy-Silanen handelt es sich um organische, nicht polymere Siliciumverbindungen, die bevorzugt aus der Gruppe der Silane mit einem, zwei oder drei Siliciumatomen ausgewählt sind.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind bevorzugt Verbindungen, die ein bis drei Silicumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt.

Kennzeichnend für die erfindungsgemäßen $C_1$-$C_6$-Alkoxy-Silane ist, dass mindestens eine $C_1$-$C_6$-Alkoxygruppe direkt an ein Siliciumatom gebunden vorliegt. Die erfindungsgemäßen $C_1$-$C_6$-Alkoxy-Silane umfassen damit mindestens eine Struktureinheit R'R"R'''Si-O-($C_1$-$C_6$-Alkyl) wobei die Reste R', R" und R''' für die drei übrigen Bindungsvalenzen des Siliciumatoms stehen.

Das oder diese an das Siliciumatom gebundenen $C_1$-$C_6$-Alkoxygruppen sind sehr reaktiv und werden in Anwesenheit von Wasser mit hoher Geschwindigkeit hydrolysiert, wobei die Reaktionsgeschwindigkeit unter anderem auch von der Anzahl der hydrolysierbaren Gruppen pro Molekül abhängt. Handelt es sich bei der hydrolysierbaren $C_1$-$C_6$-Alkoxy-Gruppe um eine Ethoxygruppe, so enthält die organische Siliciumverbindung bevorzugt eine Struktureinheit R'R"R''' Si-O-CH2-CH3. Die Reste R', R" und R''' stellen wieder die drei übrigen freien Valenzen des Siliciumatoms dar.

Im Rahmen einer ganz besonders bevorzugten Ausführungsform ist ein erfindungsgemäßes Verfahren dadurch gekennzeichnet, dass in Schritt (1) ein oder mehrere organische $C_1$-$C_6$-Alkoxy-Silane mit Wasser umgesetzt werden, die aus Silanen mit einem, zwei oder drei Siliciumatomen ausgewählt sind, wobei die organische Siliciumverbindung außerdem eine oder mehrere basische chemische Funktionen umfasst.

Bei dieser basischen Gruppe kann es sich beispielsweise um eine Aminogruppe, eine Alkylaminogruppe oder um eine Dialkylaminogruppe handeln, die bevorzugt über einen Linker mit einem Siliciumatom verbunden ist. Bevorzugt handelt es sich bei der basischen Gruppe um eine Aminogruppe, eine $C_1$-$C_6$-Alkylaminogruppe oder um eine Di($C_1$-$C_6$)alkylaminogruppe.

Ein ganz besonders bevorzugtes erfindungsgemäßes Verfahren gekennzeichnet durch das (1) Mischen eines oder mehrerer organischer $C_1$-$C_6$-Alkoxy-Silane mit Wasser,
wobei die organischem $C_1$-$C_6$-Alkoxy-Silane ausgewählt sind aus der Gruppe der Silane mit einem, zwei oder drei Siliciumatomen, und wobei die $C_1$-$C_6$-Alkoxy-Silane weiterhin eine oder mehrere basische chemische Funktionen umfassen.

Ganz besonders gute Ergebnisse konnten erhalten werden, wenn im erfindungsgemäßen Verfahren $C_1$-$C_6$-Alkoxy-Silane der Formel (I) und/oder (II) eingesetzt wurden.

In einer weiteren ganz besonders bevorzugten Ausführungsform ist ein erfindungsgemäßes Verfahren gekennzeichnet durch das

(1) Mischen eines oder mehrerer organischer $C_1$-$C_6$-Alkoxy-Silane der Formel (I) und/oder (II) mit Wasser,

R1R2N-L-Si(OR3)a(R4)b (I)

wobei

- $R_1$, $R_2$ unabhängig voneinander für ein Wasserstoffatom oder eine $C_1$-$C_6$-Alkylgruppe stehen,
- L für eine lineare oder verzweigte, zweiwertige $C_1$-$C_{20}$-Alkylengruppe steht,
- $R_3$, $R_4$ unabhängig voneinander für eine $C_1$-$C_6$-Alkylgruppe stehen,
- a, für eine ganze Zahl von 1 bis 3 steht, und
- b für die ganze Zahl 3 - a steht, und

$$(R_5O)_c(R_6)_dSi\text{-}(A)_e\text{-}[NR_7\text{-}(A')]_f\text{-}[O\text{-}(A'')]_g\text{-}[NR_8\text{-}(A''')]_h\text{-}Si(R_6')_{d'}(OR_5')_{c'} \quad (II),$$

wobei
- R5, R5‘, R5", R6, R6’ und R6" unabhängig voneinander für eine $C_1$-$C_6$-Alkylgruppe stehen,
- A, A’, A", A‴ und A″″ unabhängig voneinander für eine lineare oder verzweigte, zweiwertige $C_1$-$C_{20}$-Alkylengruppe stehen,
- $R_7$ und $R_8$ unabhängig voneinander für ein Wasserstoffatom, eine $C_1$-$C_6$-Alkylgruppe, eine Hydroxy-$C_1$-$C_6$-alkylgruppe, eine $C_2$-$C_6$-Alkenylgruppe, eine Amino-$C_1$-$C_6$-alkylgruppe oder eine Gruppierung der Formel (III) stehen,
-

$$(A'''')\text{-}Si(R_6'')_{d''}(OR_5'')_{c''} \quad (III),$$

- c, für eine ganze Zahl von 1 bis 3 steht,
- d für die ganze Zahl 3 - c steht,
- c‘ für eine ganze Zahl von 1 bis 3 steht,
- d’ für die ganze Zahl 3 - c‘ steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

Die Substituenten $R_1$, $R_2$, $R_3$, $R_4$, $R_5$, $R_5'$, $R_5''$, $R_6$, $R_6'$, $R_6''$, $R_7$, $R_8$, L, A, A’, A", A‴ und A″″ in den Verbindungen der Formel (I) und (II) sind nachstehend beispielhaft erläutert:
Beispiele für eine $C_1$-$C_6$-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine $C_2$-$C_6$-Alkenylgruppe sind Vinyl, Allyl, But-2-enyl, But-3-enyl sowie Isobutenyl, bevorzugte $C_2$-$C_6$-Alkenylreste sind Vinyl und Allyl. Bevorzugte Beispiele für eine Hydroxy-$C_1$-$C_6$-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-$C_1$-$C_6$-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In den organischen Siliciumverbindungen der Formel (I)

$$R_1R_2N\text{-}L\text{-}Si(OR_3)_a(R_4)_b \quad (I),$$

stehen die Reste $R_1$ und $R_2$ unabhängig voneinander für ein Wasserstoffatom oder eine $C_1$-$C_6$-Alkylgruppe. Ganz besonders bevorzugt stehen die Reste $R_1$ und $R_2$ beide für ein Wasserstoffatom.

Im Mittelteil der organischen Siliciumverbindung befindet sich die Struktureinheit oder der Linker -L-der für eine lineare oder verzweigte, zweiwertige $C_1$-$C_{20}$-Alkylengruppe steht. Die zweiwertige $C_1$-$C_{20}$-Alkylengruppe kann alternativ

auch als eine divalente oder zweibindige $C_1$-$C_{20}$-Alkylengruppe bezeichnet werden, womit gemeint ist, dass jede Gruppierung -L- zwei Bindungen eingehen kann.

Bevorzugt steht -L- für eine lineare, zweiwertige $C_1$-$C_{20}$-Alkylengruppe. Weiter bevorzugt steht -L- für eine lineare zweiwertige $C_1$-$C_6$-Alkylengruppe. Besonders bevorzugt steht -L- für eine Methylengruppe ($-CH_2-$), eine Ethylengruppe ($-CH_2-CH_2-$), eine Propylengruppe ($-CH_2-CH_2-CH_2-$) oder eine Butylengruppe ($-CH_2-CH_2-CH_2-CH_2-$). Ganz besonders bevorzugt steht L für eine Propylengruppe ($-CH_2-CH_2-CH_2-$).

Die erfindungsgemäßen organischen Siliciumverbindungen der Formel (I)

$$R_1R_2N\text{-}L\text{-}Si(OR_3)_a(R_4)_b \qquad (I),$$

tragen jeweils ein einem Ende die Silicium-haltige Gruppierung $-Si(OR_3)_a(R_4)_b$.

In der endständigen Struktureinheit $-Si(OR_3)_a(R_4)_b$ steht die Reste R3 und R4 unabhängig voneinander für eine $C_1$-$C_6$-Alkylgruppe, Besonders bevorzugt stehen $R_3$ und $R_4$ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Keratinbehandlungsmittel mit besonders guten Eigenschaften konnten hergestellt werden, wenn in Schritt (1) mindestens ein organisches $C_1$-$C_6$-Alkoxy-Silan der Formel (I) mit Wasser gemischt bzw. zur Reaktion gebracht wurde, bei welchem die Reste $R_3$, $R_4$ unabhängig voneinander für eine Methylgruppe oder für eine Ethylgruppe stehen.

Weiterhin konnten Färbungen mit den besten Waschechtheiten erhalten werden, wenn in Schritt (1) mindestens ein organisches $C_1$-$C_6$-Alkoxy-Silan der Formel (I) mit Wasser umgesetzt wurde, bei welchem der Rest a für die Zahl 3 steht. In diesem Fall steht der Rest b für die Zahl 0.

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane der Formel (I) mit Wasser gemischt werden, wobei

- $R_3$, $R_4$ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane der Formel (I) und/oder (II) mit Wasser gemischt bzw. zur Reaktion gebracht werden,

$$R_1R_2N\text{-}L\text{-}Si(OR_3)_a(R_4)_b \qquad (I),$$

wobei

- $R_1$, $R_2$ beide für ein Wasserstoffatom stehen, und
- L für eine lineare, zweiwertige $C_1$-$C_6$-Alkylengruppe, bevorzugt für eine Propylengruppe ($-CH_2-CH_2-CH_2-$) oder für eine Ethylengruppe ($-CH_2-CH_2-$), steht,
- $R_3$ für eine Ethylgruppe oder eine Methylgruppe steht,
- $R_4$ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Silicumverbindungen der Formel (I) sind

- (3-Aminopropyl)triethoxysilan

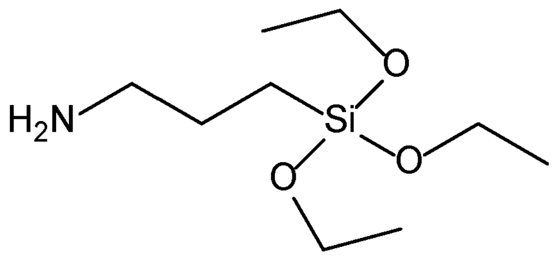

- (3-Aminopropyl)trimethoxysilan

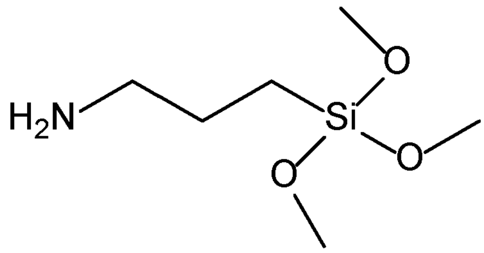

- (2-Aminoethyl)triethoxysilan

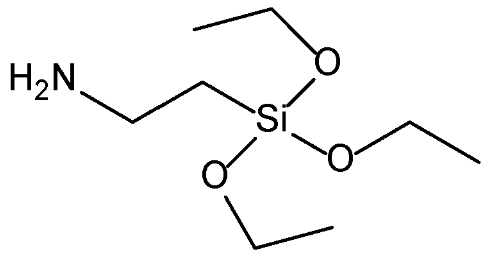

- (2-Aminoethyl)trimethoxysilan

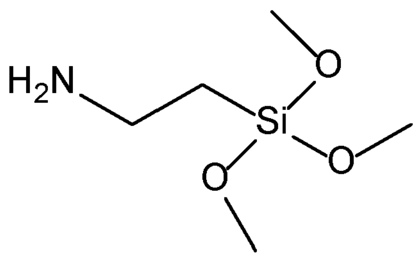

- (3-Dimethylaminopropyl)triethoxysilan

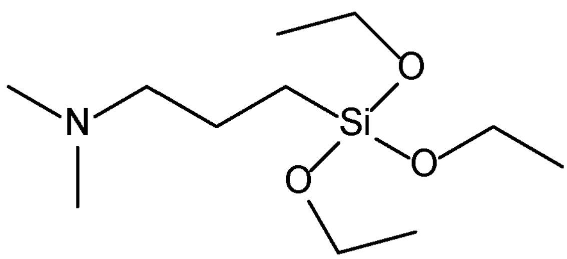

- (3-Dimethylaminopropyl)trimethoxysilan

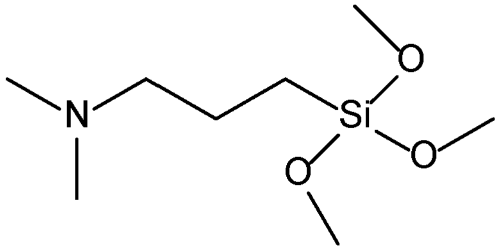

- (2-Dimethylaminoethyl)triethoxysilan.

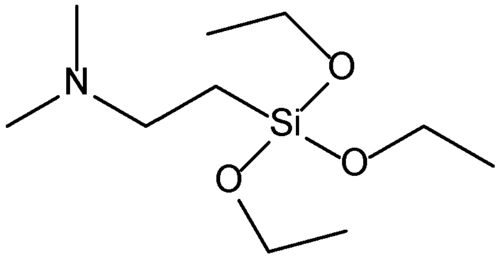

- (2-Dimethylaminoethyl)trimethoxysilan und/oder

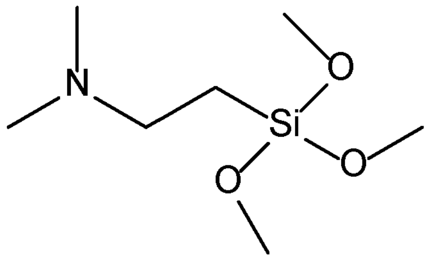

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane ausgewählt ist aus der Gruppe aus

- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1 -(2-Dimethylaminoethyl)silantriol

mit Wasser gemischt bzw. zur Reaktion gebracht werden.

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Im Rahmen einer weiteren Ausführungsform des erfindungsgemäßen Verfahrens können in Schritt (1) auch ein oder mehrere organische $C_1$-$C_6$-Alkoxy-Silane der Formel (II) mit Wasser gemischt bzw. zur Reaktion gebracht

werden,

$$(R_5O)_c(R_6)_dSi\text{-}(A)_e\text{-}[NR_7\text{-}(A')]_f\text{-}[O\text{-}(A'')]_g\text{-}[NR_8\text{-}(A''')]_h\text{-}Si(R_6')_{d'}(OR_5')_{c'} \quad (II).$$

Die erfindungsgemäßen siliciumorganischen Verbindungen der Formel (II) tragen jeweils an ihren beiden Enden die Silicium-haltigen Gruppierungen $(R_5O)_c(R_6)_dSi$- und $-Si(R_6')_{d'}(OR_5')_{c'}$.

Im Mittelteil des Moleküls der Formel (II) befinden sich die Gruppierungen $-(A)_e$- und $-[NR_7-(A')]_f$-und $-[O-(A'')]_g$- und $-[NR_8-(A''')]_h$-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine erfindungsgemäße organischen Siliciumverbindung der Formel (II) mindestens eine Gruppierung aus der Gruppe aus -(A)- und - $[NR_7-(A')]$- und -[O-(A")]- und $-[NR_8-(A''')]$-.

In den beiden endständigen Struktureinheiten $(R_5O)_c(R_6)_dSi$- und - $Si(R_6')_{d'}(OR_5')_{c'}$ stehen die Reste R5, R5', R5" unabhängig voneinander für eine $C_1$-$C_6$-Alkylgruppe. Die Reste R6, R6' und R6" stehen unabhängig voneinander für eine $C_1$-$C_6$-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Färbungen mit den besten Waschechtheiten konnten erhalten werden, wenn die Reste c und c' beide für die Zahl 3 stehen. In diesem Fall stehen d und d' beide für die Zahl 0.

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane der Formel (II) mit Wasser gemischt bzw. zur Reaktion gebracht werden,

$$(R_5O)_c(R_6)_dSi\text{-}(A)_e\text{-}[NR_7\text{-}(A')]_f\text{-}[O\text{-}(A'')]_g\text{-}[NR_8\text{-}(A''')]_h\text{-}Si(R_6')_{d'}(OR_5')_{c'} \quad (II),$$

wobei

- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die erfindungsgemäßen organischen Siliciumverbindung der Formel (IIa)

$$(R_5O)_3Si\text{-}(A)_e\text{-}[NR_7\text{-}(A')]_f\text{-}[O\text{-}(A'')]_g\text{-}[NR_8\text{-}(A''')]_h\text{-}Si(OR_5')_3 \quad (IIa).$$

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen $-(A)_e$- und $-[NR_7-(A')]_f$- und $-[O-(A'')]_g$- und $-[NR_8-(A''')]_h$- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erzielung von waschechten Färbeergebnissen erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die erfindungsgemäßen organischen Siliciumverbindung der Formel (IIb)

$$(R_5O)_c(R_6)_dSi\text{-}(A)\text{-}[NR_7\text{-}(A')]\text{-}Si(R_6')_{d'}(OR_5')_{c'} \quad (IIb).$$

Die Reste A, A', A", A‴ und A″″ stehen unabhängig voneinander für eine lineare oder verzweigte, zweiwertige $C_1$-$C_{20}$-Alkylengruppe. Bevorzugt stehen die Reste A, A', A", A‴ und A″″ unabhängig voneinander für eine lineare, zweiwertige $C_1$-$C_{20}$-Alkylengruppe. Weiter bevorzugt stehen die Reste A, A', A", A‴ und A″″ unabhängig voneinander für eine lineare zweiwertige $C_1$-$C_6$-Alkylengruppe.

Die zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung A, A', A", A‴ und A″″ zwei Bindungen eingehen kann.

Besonders bevorzugt stehen die Reste A, A', A", A‴ und A″″ unabhängig voneinander für eine Methylengruppe

($-CH_2-$), eine Ethylengruppe ($-CH_2-CH_2-$), eine Propylengruppe ($-CH_2-CH_2-CH_2-$) oder eine Butylengruppe ($-CH_2-CH_2-CH_2-CH_2-$). Ganz besonders bevorzugt stehen die Reste A, A', A", A‴ und A⁗ für eine Propylengruppe ($-CH_2-CH_2-CH_2-$).

Wenn der Rest f für die Zahl 1 steht, dann enthält die erfindungsgemäße organische Siliciumverbindung der Formel (II) eine strukturelle Gruppierung $-[NR_7-(A')]-$.

Wenn der Rest h für die Zahl 1 steht, dann enthält die erfindungsgemäße organische Siliciumverbindung der Formel (II) eine strukturelle Gruppierung $-[NR_8-(A''')]-$.

Hierbei stehen die Reste $R_7$ und $R_8$ unabhängig voneinander für ein Wasserstoffatom, eine $C_1$-$C_6$-Alkylgruppe, eine Hydroxy-$C_1$-$C_6$-alkylgruppe, eine $C_2$-$C_6$-Alkenylgruppe, eine Amino-$C_1$-$C_6$-alkylgruppe oder eine Gruppierung der Formel (III)

-

$$(A'''')-Si(R_6'')_{d''}(OR_5'')_{c''} \qquad (III).$$

Ganz besonders bevorzugt stehen die Reste R7 und R8 unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die erfindungsgemäße organische Silicumverbindung die Gruppierung $[NR_7-(A')]$, aber nicht die Gruppierung $-[NR_8-(A''')]$. Steht nun der Rest R7 für eine Gruppierung der Formel (III), so enthält das VorbehandlungsMittel (a) eine organische Silicumverbindung mit 3 reaktiven Silan-Gruppen.

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane der Formel (II) mit Wasser zur Reaktion gebracht werden

$$(R_5O)_c(R_6)_dSi-(A)_e-[NR_7-(A')]_f-[O-(A'')]_g-[NR_8-(A''')]_h-Si(R_6')_{d'}(OR_5')_{c'} \qquad (II),$$

wobei

- e und f beide für die Zahl 1 stehen,
- g und h beide für die Zahl 0 stehen,
- A und A‘ unabhängig voneinander für eine lineare, zweiwertige $C_1$-$C_6$-Alkylengruppe stehen
  und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane der Formel (II) mit Wasser gemischt bzw. zur Reaktion gebracht werden, wobei

- e und f beide für die Zahl 1 stehen,
- g und h beide für die Zahl 0 stehen,
- A und A‘ unabhängig voneinander für eine Methylengruppe ($-CH_2-$), eine Ethylengruppe ($-CH_2-CH_2-$) oder eine Propylengruppe ($-CH_2-CH_2-CH_2$) stehen,
  und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der erfindungsgemäßen Aufgabenstellung gut geeignete organische Silicumverbindungen der Formel (II) sind

- 3-(Trimethoxysilyl)-N-[3-(trimethoxysilyl)propyl]-1-propanamin

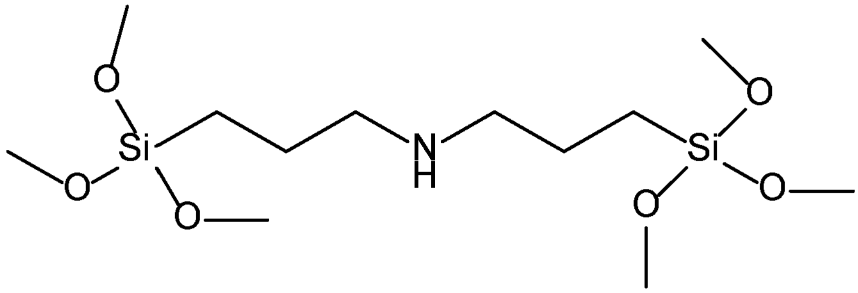

- 3-(Triethoxysilyl)-N-[3-(triethoxysilyl)propyl]-1-propanamin

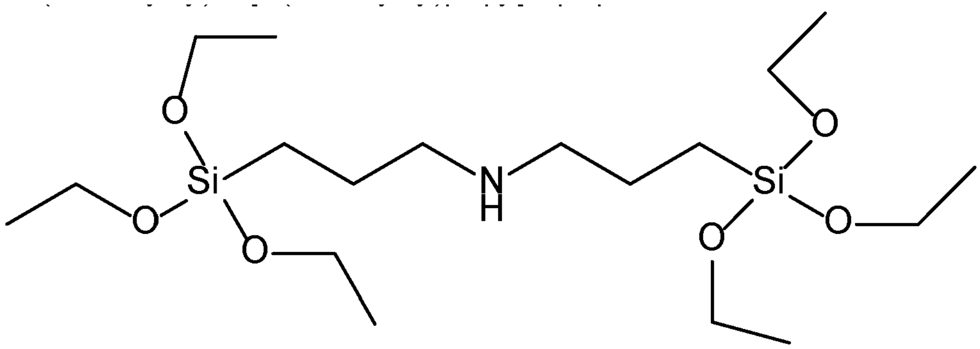

- N-Methyl-3-(trimethoxysilyl)-N-[3-(trimethoxysilyl)propyl]-1-propanamin

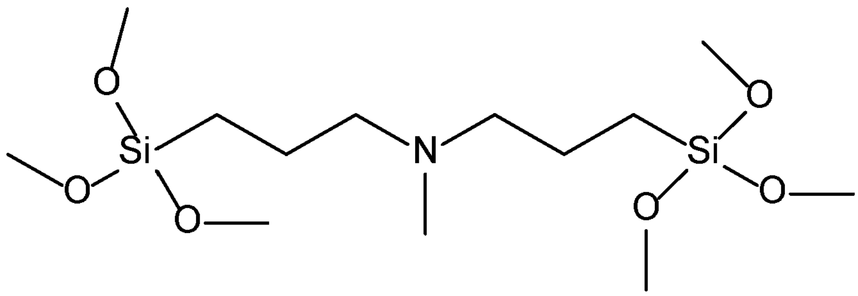

- N-Methyl-3-(triethoxysilyl)-N-[3-(triethoxysilyl)propyl]-1-propanamin

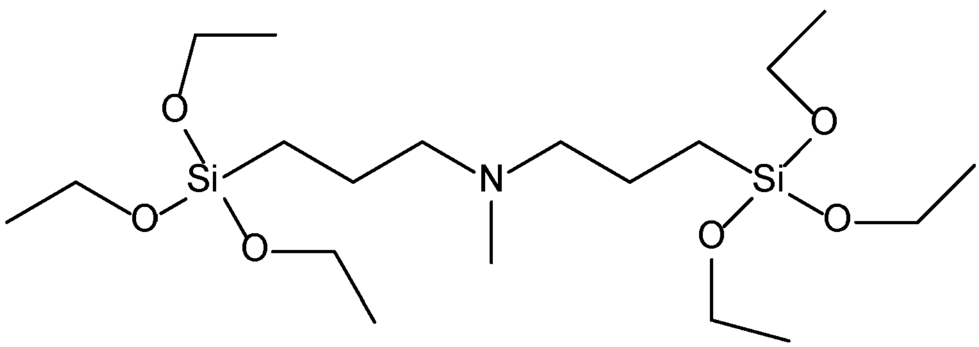

- 2-[Bis[3-(trimethoxysilyl)propyl]amino]-ethanol

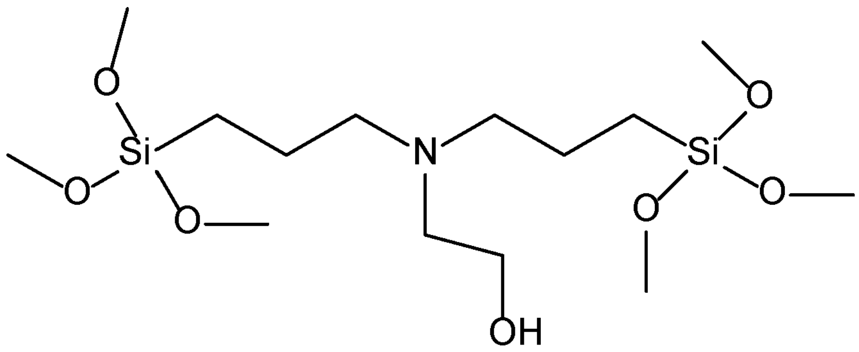

- 2-[Bis[3-(triethoxysilyl)propyl]amino]-ethanol

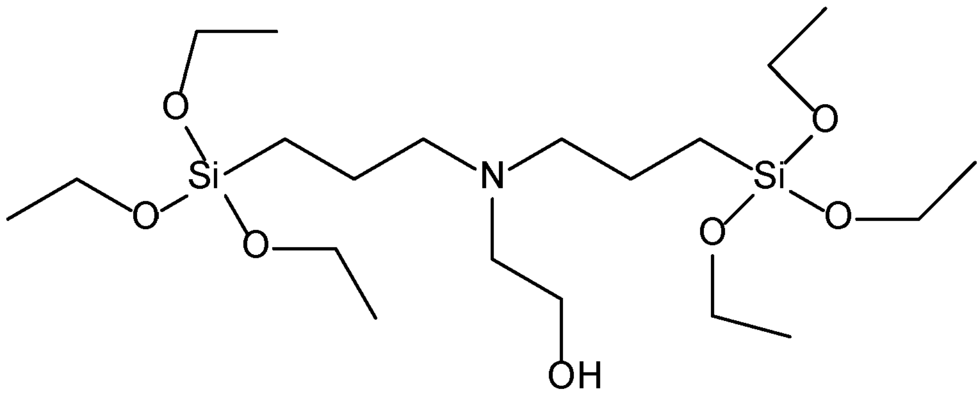

- 3-(Trimethoxysilyl)-N,N-bis[3-(trimethoxysilyl)propyl]-1-propanamin

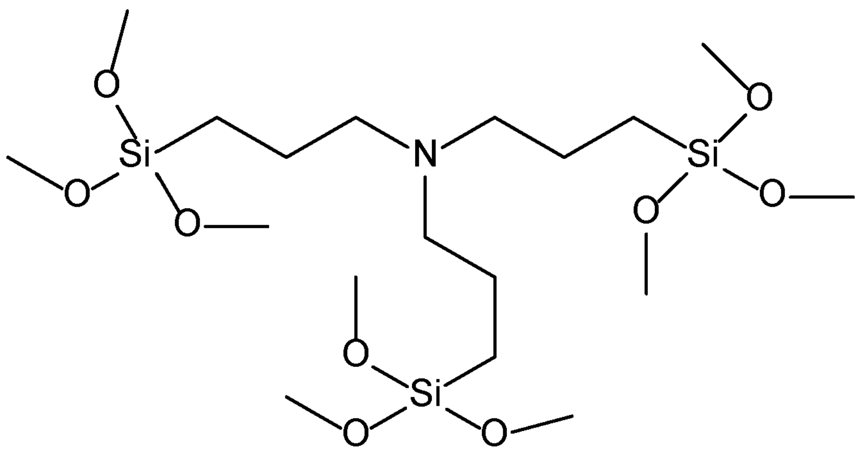

- 3-(Triethoxysilyl)-N,N-bis[3-(triethoxysilyl)propyl]-1-propanamin

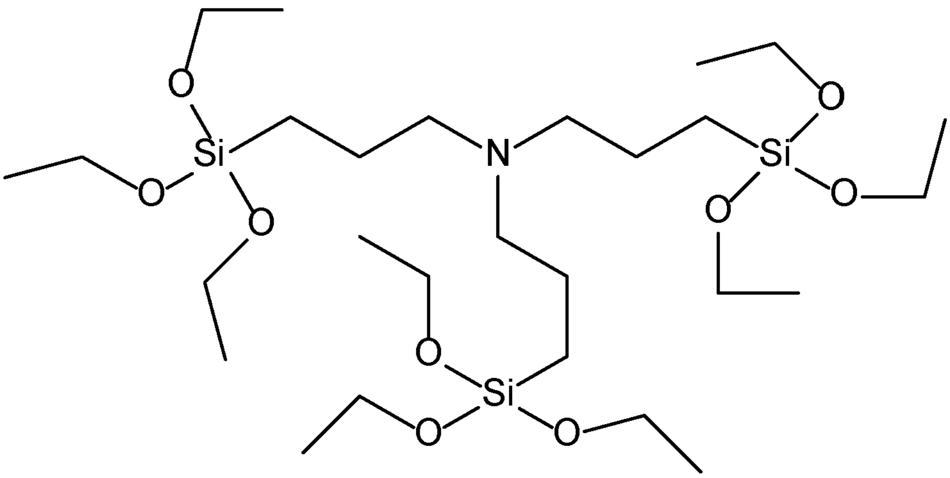

- N1,N1-Bis[3-(trimethoxysilyl)propyl]-1,2-ethanediamin,

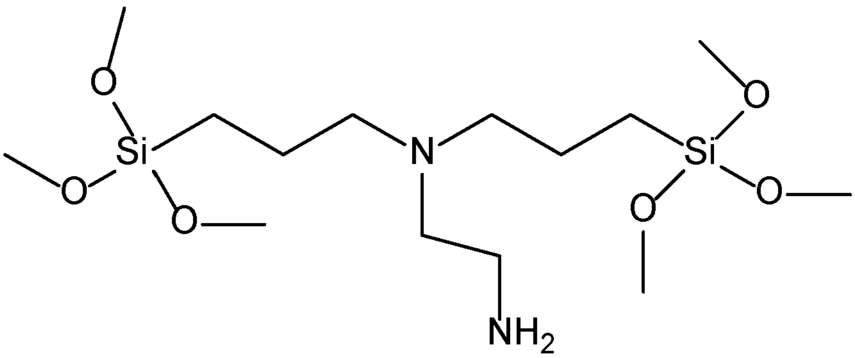

- N1,N1-Bis[3-(triethoxysilyl)propyl]-1,2-ethanediamin,

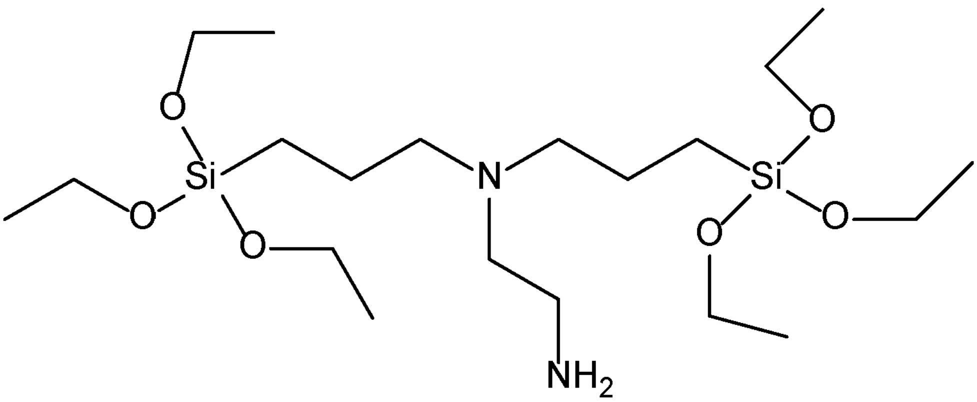

- N,N-Bis[3-(trimethoxysilyl)propyl]-2-propen-1-amin

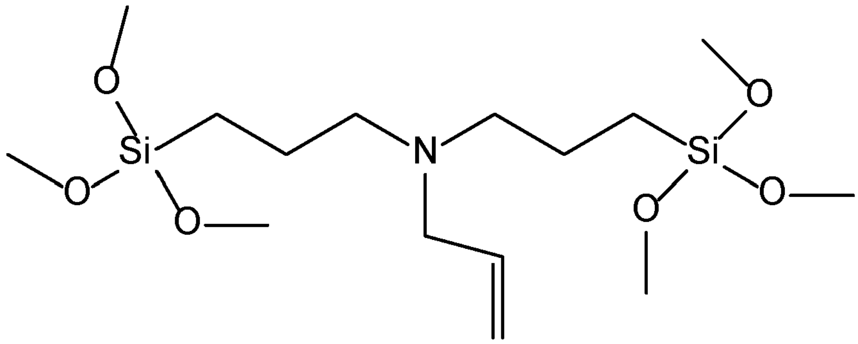

- N,N-Bis[3-(triethoxysilyl)propyl]-2-propen-1-amin

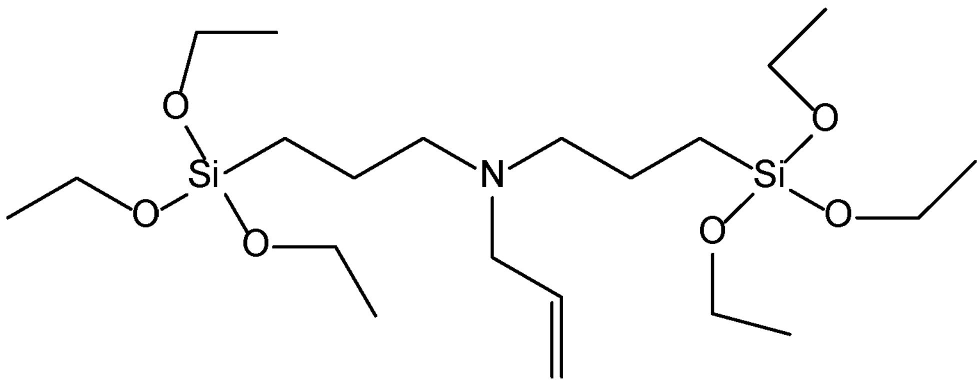

Die vorgenannten organische Siliciumverbindung der Formel (II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane der Formel (II), die ausgewählt sind aus der Gruppe aus

- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin,

mit Wasser zur Reaktion gebracht bzw. mit Wasser gemischt werden.

In weiteren Färbeversuchen hat es sich ebenfalls als ganz besonders vorteilhaft herausgestellt, wenn im erfindungsgemäßen Verfahren mindestens ein organisches $C_1$-$C_6$-Alkoxy-Silan der Formel (IV) eingesetzt wurde

$$R_9Si(OR_{10})_k(R_{11})_m \qquad (IV).$$

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere hydrolysierbare Gruppen pro Molekül umfasst.

Das bzw. die organischen Siliciumverbindungen der Formel (IV) können auch als Silane vom Typ der Alkyl-$C_1$-$C_6$-alkoxy-silane bezeichnet werden,

$$R_9Si(OR_{10})_k(R_{11})_m \qquad (IV),$$

wobei

- $R_9$ für eine $C_1$-$C_{12}$-Alkylgruppe steht,
- $R_{10}$ für eine $C_1$-$C_6$-Alkylgruppe steht,
- $R_{11}$ für eine $C_1$-$C_6$-Alkylgruppe steht
- k für eine ganze Zahl von 1 bis 3 steht, und
- m für die ganze Zahl 3 - k steht.

In einer weiteren Ausführungsform ist ein besonders bevorzugtes erfindungsgemäßes Verfahren gekennzeichnet durch das

(1) Mischen eines oder mehrerer organischer $C_1$-$C_6$-Alkoxy-Silane der Formel (IV) mit Wasser,

$$R_9Si(OR_{10})_k(R_{11})_m \qquad (IV),$$

wobei

- $R_9$ für eine $C_1$-$C_{12}$-Alkylgruppe steht,
- $R_{10}$ für eine $C_1$-$C_6$-Alkylgruppe steht,
- $R_{11}$ für eine $C_1$-$C_6$-Alkylgruppe steht
- k für eine ganze Zahl von 1 bis 3 steht, und
- m für die ganze Zahl 3 - k steht.

In den organischen $C_1$-$C_6$-Alkoxy-Silanen der Formel (IV) steht der Rest $R_9$ für eine $C_1$-$C_{12}$-Alkylgruppe. Diese $C_1$-$C_{12}$-Alkylgruppe ist gesättigt und kann linear oder verzweigt sein. Bevorzugt steht R9 für eine lineare $C_1$-$C_8$-Alkylgruppe. Bevorzugt steht $R_9$ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht $R_9$ für eine Methylgruppe, eine Ethylgruppe oder eine n-Octylgruppe.

In den organischen Siliciumverbindungen der Formel (IV) steht der Rest $R_{10}$ für eine $C_1$-$C_6$-Alkylgruppe. Besonders bevorzugt steht R10 für eine Methylgruppe oder für eine Ethylgruppe.

In den organischen Siliciumverbindungen der Formel (IV) steht der Rest $R_{11}$ für eine $C_1$-$C_6$-Alkylgruppe. Besonders bevorzugt steht R11 für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Färbungen mit den besten Waschechtheiten konnten erhalten werden, wenn bei der Herstellung der erfindungsgemäßen Zubereitung mindestens eine organische Siliciumverbindung der Formel (IV) eingesetzt wurde, bei welcher der Rest k für die Zahl 3 steht. In diesem Fall steht der Rest m für die Zahl 0.

Zur Lösung der erfindungsgemäßen Aufgabenstellung besonders gut geeignete organische Silicumverbindungen der Formel (IV) sind

- Methyltrimethoxysilan

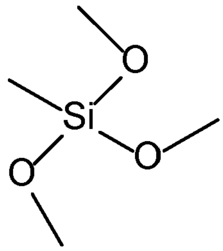

- Methyltriethoxysilan

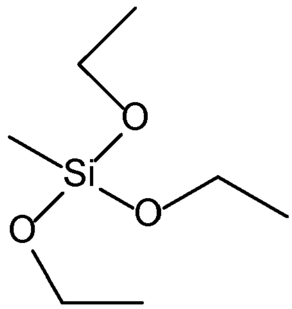

- Ethyltrimethoxysilan

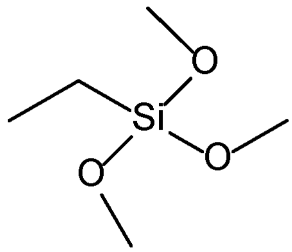

- Ethyltriethoxysilan

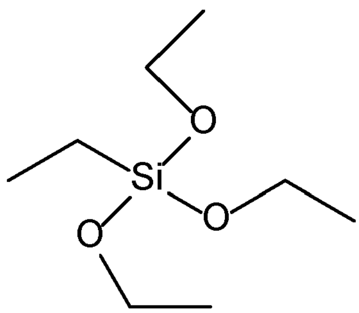

- n-Hexyltrimethoxysilan (auch bezeichnet als Hexyltrimethoxysilan)

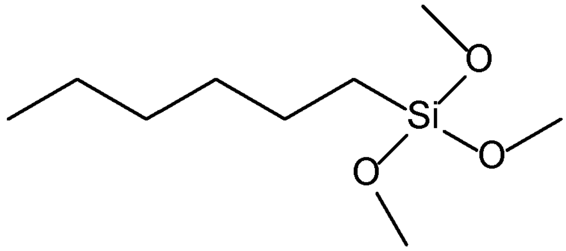

- n-Hexyltriethoxysilan (auch bezeichnet als Hexyltriethoxysilan)

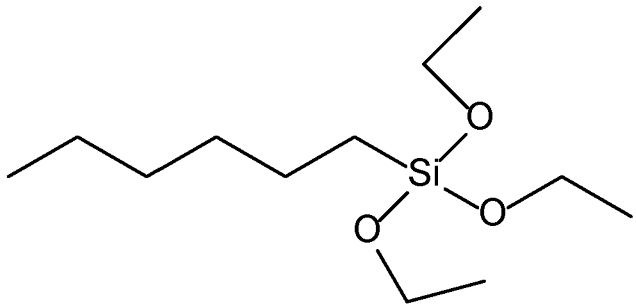

- n-Octyltrimethoxysilan (auch bezeichnet als Octyltrimethoxysilan)

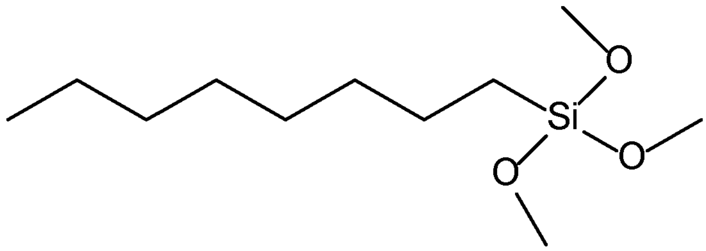

- n-Octyltriethoxysilan (auch bezeichnet als Octyltriethoxysilan)

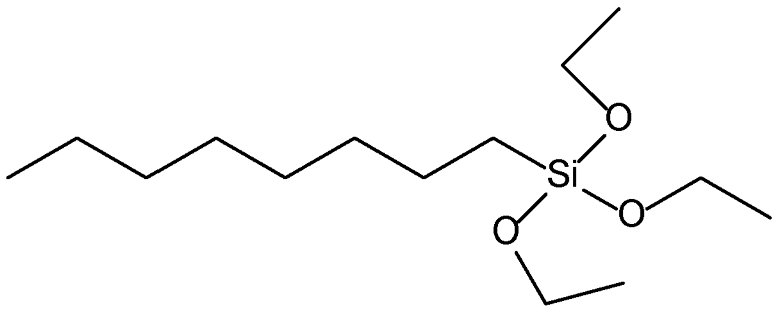

- n-Dodecyltrimethoxysilan (auch bezeichnet als Dodecyltrimethoxysilan) und/oder

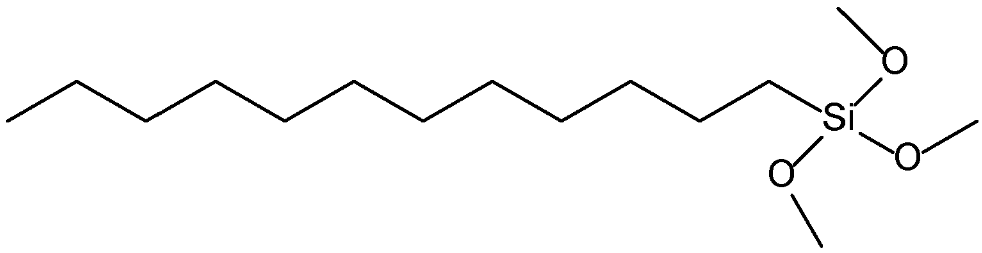

- n-Dodecyltriethoxysilan (auch bezeichnet als Dodecyltriethoxysilan).

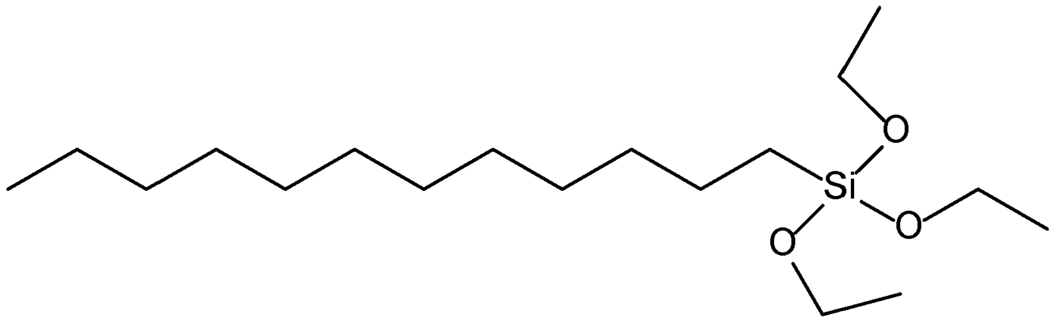

In einer weiteren bevorzugten Ausführungsform ist ein erfindungsgemäßes Verfahren dadurch gekennzeichnet, dass in Schritt (1) ein oder mehrere organische $C_1$-$C_6$-Alkoxy-Silane der Formel (IV), die ausgewählt sind aus der Gruppe aus

- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan und/oder
- Dodecyltriethoxysilan,

mit Wasser gemischt bzw. mit Wasser zur Reaktion gebracht werden.

Weiterhin hat es sich als ganz besonders bevorzugt herausgestellt, in Schritt (1) des erfindungsgemäßen Verfahrens mindestens ein organisches $C_1$-$C_6$-Alkoxy-Silane der Formel (I) und mindestens ein organisches $C_1$-$C_6$-Alkoxy-Silane der Formel (IV) mit Wasser zu mischen.

Das erfindungsgemäße Verfahren kann in einem hierfür geeigneten Reaktionsgefäß oder auch Reaktor durchgeführt werden. Abhängig von der gewünschten Ansatzgröße sind hierfür verschiedene Modelle aus dem Stand der Technik bekannt und kommerziell erwerbbar.

So kann die Reaktion der organischen $C_1$-$C_6$-Alkoxy-Silane mit Wasser beispielsweise in einem Reaktionsgefäß oder einem Reaktor, bevorzugt in einem Doppelwandreaktor, einem Reaktor mit externem Wärmeaustauscher, einem Rohrreaktor, einem Reaktor mit Dünnfilm-Verdampfer, einem Reaktor mit Fallfilmverdampfer und/oder einem Reaktor mit angeschlossenem Kondensator durchgeführt werden.

In einer weiteren besonders bevorzugten Ausführungsform ist ein erfindungsgemäßes Verfahren gekennzeichnet durch das

(1) Mischen eines oder mehrerer organischer $C_1$-$C_6$-Alkoxy-Silane mit Wasser in einem Reaktionsgefäß oder einem Reaktor, bevorzugt in einem Doppelwandreaktor, einem Reaktor mit externem Wärmeaustauscher, einem Rohrreaktor, einem Reaktor mit Dünnfilm-Verdampfer, einem Reaktor mit Fallfilmverdampfer und/oder einem Reaktor mit angeschlossenem Kondensator.

Ein für kleinere Ansätze sehr gut geeignetes Reaktionsgefäß ist beispielsweise ein üblicherweise für chemische Reaktionen eingesetzter Glaskolben mit einem Fassungsvermögen von 1 Liter, 3 Litern oder 5 Litern handeln, beispielsweise ein 3-Liter Ein- oder Mehrhalskolben mit Schliffen.

Als Reaktor wird ein abgegrenzter Raum (Behältnis, Behälter) bezeichnet, der speziell dafür konstruiert und hergestellt wurde, um darin unter definierten Bedingungen bestimmte Reaktionen ablaufen lassen und steuern zu können.

Für größere Ansätze hat es sich als vorteilhaft herausgestellt, die Reaktion in Reaktoren aus Metall durchzuführen. Typische Reaktoren können beispielsweise eine Füllmenge von 10-Litern, 20-Litern oder 50-Litern umfassen. Größere Reaktoren für den Produktionsbereich können auch Füllmengen von 100-Litern, 500-Litern oder 1000-Litern umfassen.

Doppelwandreaktoren besitzen zwei Reaktor-Hüllen bzw. Reaktor-Wandungen, wobei in dem zwischen beiden

Wandungen befindlichen Bereich eine Temperier-Flüssigkeit zirkulieren kann. Diese ermöglicht eine besonders gute Einstellung der Temperatur auf die benötigten Werte.

Als besonders gut geeignet hat sich auch der Einsatz von Reaktoren, insbesondere DoppelwandReaktoren mit vergrößerter Wärme-Austauschfläche erwiesen, wobei der Wärmeaustauch hierbei entweder durch interne Einbauten oder auch durch Einsatz eines externen Wärmeaustauschers erfolgen kann.

Entsprechende Reaktoren sind beispielsweise Laborreaktoren der Firma IKA. In diesem Zusammenhang können die Modelle "LR-2.ST" oder das Modell "magic plant" genannt werden.

Weitere einsetzbare Reaktoren sind Reaktoren mit Dünnfilm-Verdampfer, da auf diesem Wege eine sehr gute Wärmeabfuhr und damit eine besonders exakte Temperierung vorgenommen werden kann. Dünnfilm-Verdampfer werden alternativ auch als Dünnschicht-Verdampfer bezeichnet. Dünnschicht-Verdampfer können beispielsweise von der Asahi Glassplant Inc. kommerziell erworben werden.

Bei Reaktoren mit Fallfilmverdampfer findet die Verdampfung im Allgemeinen in einem Rohr statt, d.h. die zu verdampfende Flüssigkeit (d.h. in diesem Fall die in Schritt (2) zu entfernenden $C_1$-$C_6$-Alkohole) fließen als zusammenhängender Flüssigkeitsfilm. Auch Reaktoren mit Fallfilmverdampfer sind von verschiedenen Anbietern kommerziell erhältlich.

Die in Schritt (1) stattfindende Reaktion der organischen $C_1$-$C_6$-Alkoxy-Silane mit Wasser kann auf verschiedenen Wegen stattfinden. Die Reaktion startet, sobald die $C_1$-$C_6$-Alkoxy-Silane mit Wasser durch Vermischen in Kontakt kommen. Eine Möglichkeit ist es, die gewünschte Wassermenge im Reaktionsgefäß oder Reaktor vorzulegen und im Anschluss daran dass oder die $C_1$-$C_6$-Alkoxy-Silane hinzuzufügen.

In einer weiteren Ausführungsform ist es auch möglich, zunächst das oder die organischen $C_1$-$C_6$-Alkoxy-Silane im Reaktionsgefäß bzw. Reaktor vorzulegen und dann die gewünschte Menge Wasser hinzuzugeben.

Sobald $C_1$-$C_6$-Alkoxy-Silane und Wasser in Kontakt kommen, findet eine exotherme Hydrolyse-Reaktion gemäß folgendem Schema statt (Reaktionsschema am Beispiel von 3-Aminopropyltriethoxysilan):

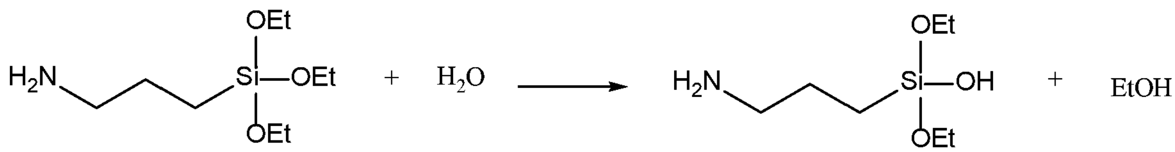

In Abhängigkeit von der Anzahl der hydrolysierbaren $C_1$-$C_6$-Alkoxygruppen pro Silan-Molekül kann die Hydrolysereaktion auch mehrfach pro eingesetztem $C_1$-$C_6$-Alkkoxy-Silan stattfinden:

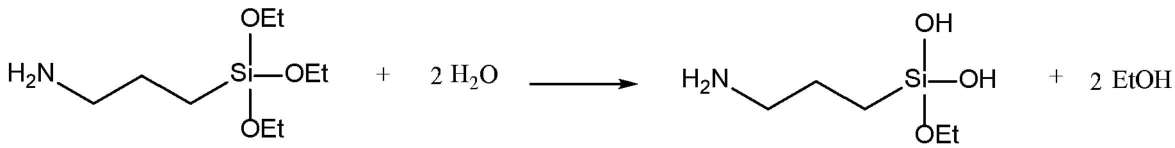

bzw.

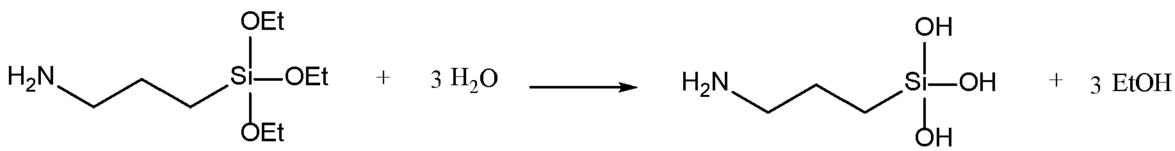

Da die Hydrolyse-Reaktion exotherm ist, hat es sich als besonders vorteilhaft herausgestellt, das Reaktionsgemisch aus Wasser und organischen $C_1$-$C_6$-Alkoxy-Silane zur verbesserten Wärmeabfuhr zu rühren bzw. zu durchmischen.

Das Wasser kann kontinuierlich, in Teilmengen oder direkt als Gesamtmenge zugegeben werden. Um eine ausreichende Temperaturkontrolle zu gewährleisten, wird die Reaktionsmischung bevorzugt gekühlt und/oder die Zugabemenge und -geschwindigkeit des Wassers angepasst. Je nach Menge der eingesetzten Silane kann die Zugabe und Reaktion über einen Zeitraum von 2 Minuten bis zu 72 Stunden erfolgen.

Zur Herstellung von Mitteln, die ein besonders gutes Coating auf dem Keratinmaterial erzeugen, hat es sich als explizit ganz besonders bevorzugt herausgestellt, dass Wasser in einer unterstöchiometrischen Menge in Schritt (1) einzusetzen. In diesem Fall liegt die Einsatzmenge an Wasser unterhalb der Menge, die theoretisch erforderlich wäre, um alle vorhandenen hydrolysierbaren $C_1$-$C_6$-Alkoxy-Gruppen an den Si-Atomen, d.h. die Alkoxysilangruppen, zu hydrolysieren. Ganz besonders bevorzugt ist demzufolge die partielle Hydrolyse der organischem $C_1$-$C_6$-Alkoxy-Silane.

Das stöchiometrische Verhältnis von Wasser zu den organischem $C_1$-$C_6$-Alkoxy-Silanen kann über den Anteil an Stoffmengenäquivalenten Wasser (S-W) definiert werden, diese berechnen sich nach der folgenden Formel:

$$\text{S-W} = \frac{mol(Wasser)}{mol(Silane)\ x\ n(Alkoxy)}$$

mit

S-W= Stoffmengenäquivalent Wasser
mol(Wasser) = eingesetzte Molmenge Wasser
mol(Silane) = Gesamtmolmenge der in der Reaktion eingesetzten $C_1$-$C_6$-Alkoxy-Silane
n(Alkoxy) = Anzahl der $C_1$-$C_6$-Alkoxygruppen pro $C_1$-$C_6$-Alkoxy-Silan

Mit anderen Worten gibt das Stoffmengenäquivalent an Wasser das molare Verhältnis aus der eingesetzten Molmenge an Wasser zu der gesamten Molanzahl an hydrolysierbaren $C_1$-$C_6$-Alkoxygruppen an, die sich an den eingesetzten $C_1$-$C_6$-Alkoxysilanen befinden.

In einer weiteren ganz besonders bevorzugten Ausführungsform ist ein erfindungsgemäßes Verfahren gekennzeichnet durch das

(1) Mischen der organischen $C_1$-$C_6$-Alkoxy-Silane mit 0,10 bis 0,80 Stoffmengenäquivalenten Wasser (S-W), bevorzugt von 0,15 bis 0,70, weiter bevorzugt von 0,20 bis 0,60 und ganz besonders bevorzugt von 0,25 bis 0,50 Stoffmengenäquivalenten Wasser,
wobei die Stoffmengenäquivalente Wasser sich berechnen nach der Formel

$$\text{S-W} = \frac{mol(Wasser)}{mol(Silane)\ x\ n(Alkoxy)}$$

mit

S-W= Stoffmengenäquivalent Wasser
mol(Wasser) = eingesetzte Molmenge Wasser
mol(Silane) = Gesamtmolmenge der in der Reaktion eingesetzten $C_1$-$C_6$-Alkoxy-Silane
n(Alkoxy) = Anzahl der $C_1$-$C_6$-Alkoxygruppen pro $C_1$-$C_6$-Alkoxy-Silan

Beispiel

In einem Reaktionsgefäß wurden 20,0 g 3-Aminopropyltriethoxsilan ($C_9H_{23}NO_3Si$ = 221,37 g/mol) und 50,0 g Methyltrimethoxysilan ($C_4H_{12}O_3Si$ = 136,22 g/mol) miteinander vermischt.

20,0 g 3-Aminopropyltriethoxsilan = 0,0903 mol (3 hydrolysierbare Alkoxygruppen pro Molekül) 50,0 g Methyltrimethoxysilan = 0,367 mol (3 hydrolysierbare Alkoxygruppen pro Molekül)

Anschließend wurden unter Rühren 10,0 g Wasser (18,015 g/mol) hinzugetropft. 10,0 g Wasser = 0,555 mol

Stoffmengenäquivalente Wasser = 0,555 mol / [ (3 x 0,090 mol) + (3 x 0,367 mol) ] = 0,40

In dieser Reaktion wurden die eingesetzten $C_1$-$C_6$-Alkoxysilane mit 0,40 Stoffmengenäquivalenten Wasser umgesetzt.

Für die Herstellung besonders leistungsstarker Keratinbehandlungsmittel hat sich bei Schritt (1) die Einhaltung spezieller Temperaturbereiche als ganz besonders vorteilhaft herausgestellt.

In diesem Zusammenhang konnte gefunden werden, dass eine Mindest-Temperatur von 20 °C in Schritt (1) besonders gut geeignet ist, um die Hydrolyse mit ausreichend hoher Geschwindigkeit ablaufen zu lassen und eine effiziente Reaktionsführung zu gewährleisten.

Auf der anderen Seite sollte jedoch eine Erwärmung des Reaktionsgemisches auf Temperaturen über 70 °C

vermieden werden. Erfolgt die Herstellung bei zu hohen Temperaturen, so findet vermutlich eine an dieser Stelle unerwünschte oder zu starke Polymerisierung bzw. Kondensationsreaktion statt, die dazu führt, dass sich bei der späteren Anwendung des Mittels auf dem Keratinmaterial kein an dem Keratin anhaftender Film mehr ausbilden kann. Bei Anwendung eines bei zu hohen Temperaturen hergestelltes Mittels in einem Färbeverfahren konnten somit keine ausreichend hohen Farbintensitäten mehr erzielt werden.

Aus diesen Gründen sollte die Reaktion des oder der organischer $C_1$-$C_6$-Alkoxy-Silane mit Wasser in Schritt (1) des Verfahrens bei einer Temperatur von 20 bis 70 °C durchgeführt werden.

Der hier angegebene Temperaturbereich bezieht sich auf die Temperatur, auf die das Gemisch aus $C_1$-$C_6$-Alkoxy-Silane und Wasser eingestellt sein sollte. Gemessen werden kann diese Temperatur beispielsweise durch ein in diese Mischung hineinragendes, geeichtes Thermometer. Bevorzugt erfolgt die Reaktion eines oder mehrerer organischer $C_1$-$C_6$-Alkoxy-Silane mit Wasser bei einer Temperatur von 20 °C bis 70 °C, bevorzugt von 20 bis 65 °C, weiter bevorzugt von 20 bis 60 °C, noch weiter bevorzugt von 20 bis 55 °C, noch weiter bevorzugt von 20 bis 50 °C und ganz besonders bevorzugt von 20 bis 45 °C.

In einer weiteren ganz besonders bevorzugten Ausführungsform ist ein erfindungsgemäßes Verfahren gekennzeichnet durch das

(1) Mischen eines oder mehrerer organischer $C_1$-$C_6$-Alkoxy-Silane mit Wasser bei einer Temperatur von 20 °C bis 70 °C, bevorzugt von 20 bis 65 °C, weiter bevorzugt von 20 bis 60 °C, noch weiter bevorzugt von 20 bis 55 °C, noch weiter bevorzugt von 20 bis 50 °C und ganz besonders bevorzugt von 20 bis 45 °C.

Die Einstellung der bevorzugten und besonders bevorzugten Temperaturbereiche kann durch Temperieren des Reaktionsgefäßes oder Reaktors erfolgen. Beispielsweise kann das Reaktionsgefäß oder der Reaktor von außen mit einem Temperierbad umgeben werden, bei dem es sich beispielsweise um ein Wasserbad oder um Silikonölbad handeln kann.

Wird die Reaktion in einem Doppelwandreaktor durchgeführt, so kann auch eine temperierte Flüssigkeit durch den Raum geleitet werden, der durch die beiden Wandungen gebildet wird und der den Reaktionsraum umgibt.

Es kann ferner bevorzugt sein, dass kein aktives Erwärmen der Reaktionsmischung erfolgt und eine etwaige Erhöhung der Temperatur über die Umgebungstemperatur nur durch die Exothermie der Hydrolyse in Schritt (1) bewirkt wird. Erwärmt der exotherme Reaktionsablauf die Reaktionsmischung in Schritt (1) zu stark, muss wiederum gekühlt werden.

Die Reaktion der organischer $C_1$-$C_6$-Alkoxy-Silane mit Wasser findet bevorzugt bei Normaldruck, d.h. bei einem Druck von 1013 mbar (1013 hPa) statt.

<u>Entfernung der in Schritt (1) freigesetzten $C_1$-$C_6$-Alkohole aus dem Reaktionsgemisch</u>

Schritt (2) des erfindungsgemäßen Verfahrens umfasst die partielle oder vollständige Entfernung der durch die Reaktion in Schritt (1) freigesetzten $C_1$-$C_6$-Alkohole aus dem Reaktionsgemisch.

Hierbei hat es sich als erfindungswesentlich herausgestellt, die $C_1$-$C_6$-Alkohole so vollständig wie möglich aus dem Reaktionsgemisch zu entfernen, so dass der Gesamtgehalt an $C_1$-$C_6$-Alkoholen in der finalen Zubereitung, nach Schritt (4) erhaltenen Zubereitung unterhalb eines Wert von 8,0 Gew.- % liegt.

In vergleichenden Färbeversuchen konnte gefunden werden, dass die Anwesenheit von zu hohen Restmengen an $C_1$-$C_6$-Alkoholen sich bei der späteren Anwendung der Zubereitung auf dem Keratinmaterial negativ auf die Färbeleistung und Farbintensität auswirkt.

Wie zuvor beschrieben werden bei der Hydrolyse der $C_1$-$C_6$-Alkoxysilane die entsprechenden $C_1$-$C_6$-Alkohole freigesetzt, die nun in Schritt (2) aus dem Reaktionsgemisch entfernt und auf diese Weise dem Reaktionsgleichgewicht entzogen werden können.

Da die $C_1$-$C_6$-Alkohole erst nach ihrer in Schritt (1) stattfindenden Freisetzung aus dem Reaktionsgemisch entfernt werden können, erfolgt Schritt (2) des Verfahrens bevorzugt nach dem Schritt (1). Hierbei kann die Entfernung der $C_1$-$C_6$-Alkohole entweder direkt nach der Hydrolyse in Schritt (1) erfolgen. Alternativ kann aber auch zunächst ein kosmetischer Inhaltstoff (entsprechend Schritt (3) des erfindungsgemäßen Verfahrens) hinzugefügt werden und die Entfernung der $C_1$-$C_6$-Alkohole (Schritt (2)) im Anschluss daran vorgenommen werden.

Alternativ kann in verschiedenen Ausführungsformen die Durchführung des Schritts (2) auch gleichzeitig mit der Hydrolyse in Schritt (1) erfolgen. In derartigen Ausführungsformen wird bereits vor Zugabe des Wassers, bei Start der Zugabe oder nachdem 5-20 Gew.-% der geplanten Gesamtmenge des Wassers zugegeben wurden, mit der Entfernung der $C_1$-$C_6$-Alkohole begonnen, d.h. die Destillation - gegebenenfalls unter Druckreduktion - gestartet.

Bedingt durch die Entfernung der $C_1$-$C_6$-Alkohole wird das Reaktionsgleichgewicht zugunsten einer Kondensationsreaktion verschoben, bei der die an den (partiell) hydrolysierten $C_1$-$C_6$-Alkoxysilanen befindlichen Si-OH Gruppen unter Wasserabspaltung mit weiteren Si-OH Gruppen oder aber mit weiteren $C_1$-$C_6$-Alkoxy-Silangruppen reagieren

können.

Eine derartige Reaktion kann beispielsweise gemäß dem folgenden Schema ablaufen:

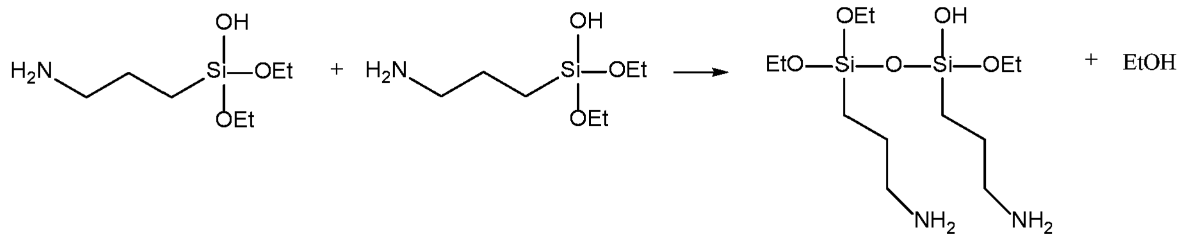

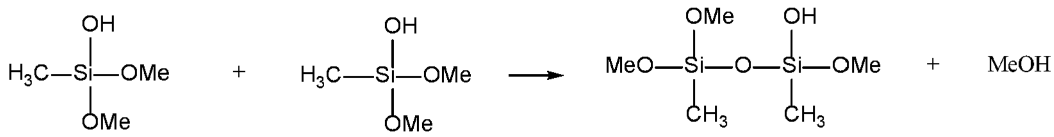

An der Kondensationsreaktion können sowohl partiell hydrolysierte als auch vollständig hydrolysierte $C_1$-$C_6$-Alkoxysilane teilnehmen, die eine Kondensation mit noch nicht abreagierten, partiell oder auch vollständig hydrolysierten $C_1$-$C_6$-Alkoxysilanen durchlaufen.

Im obigen beispielhaften Reaktionsschema ist die Kondensation zu einem Dimer gezeigt, jedoch ist auch die Kondensation zu Oligomeren mit mehreren Silan-Atomen möglich und auch bevorzugt.

Darüber hinaus ist auch eine Kondensation von $C_1$-$C_6$-Alkoxysilanen verschiedener Strukturen möglich, so können beispielsweise die $C_1$-$C_6$-Alkoxysilane der Formel (I) mit den $C_1$-$C_6$-Alkoxy-silanen der Formel (IV) kondensieren.

Wenn die freigesetzten $C_1$-$C_6$-Alkohole in Schritt (2) des Verfahrens nicht in ausreichendem Ausmaß aus dem Reaktionsgemisch entfernt werden, so kann sich das oben gezeigte Reaktionsgleichgewicht vermutlich wieder zurück auf die Seite der monomeren Verbindungen verschieben. Durch diese Rückreaktion wird die Ausbildung der oligomeren Silan-Kondensaten mit ausreichend hohem Molekulargewicht verhindert, was bei späterer Anwendung der Formulierungen auf dem Keratinmaterial in zu niedrigen Farbintensitäten und zu einer schlechteren Haltbarkeit des gebildeten Films bzw. Coatings führt.

Im erfindungsgemäßen Verfahren wird eine möglichst vollständige Entfernung der freigesetzten $C_1$-$C_6$-Alkohole vorgenommen. Die restlose Entfernung aller $C_1$-$C_6$-Alkohole lässt sich nur schwer realisieren, da kleine Reste an $C_1$-$C_6$-Alkoholen immer im Reaktionsgemisch verbleiben werden, insbesondere wenn das Reaktionsgemisch nicht zu stark erwärmt werden soll. Es ist jedoch erfindungswesentlich, in Schritt (2) des Verfahrens alle $C_1$-$C_6$-Alkohole so vollständig zu entfernen, dass der Gesamtgehalt an $C_1$-$C_6$-Alkoholen in der finalen, nach Schritt (4) erhaltenen Zubereitung - bezogen auf das Gesamtgewicht der Zubereitung - unterhalb von 10,0 Gew.-% gehalten werden kann.

Das Ausmaß der Kondensationsreaktion wird durch die in Schritt (1) zugegebene Menge an Wasser mitbestimmt. Bevorzugt wird die Menge an Wasser so bemessen, dass die Kondensation eine Teilkondensation ist, wobei "Teilkondensation" bzw. "partielle Kondensation" in diesem Kontext bedeutet, dass nicht alle kondensierbaren Gruppen der vorgelegten Silane miteinander reagieren, so dass die entstehende organische Siliciumverbindung pro Molekül im Mittel jeweils noch mindestens eine hydrolysierbare/kondensierbare Gruppe aufweist.

Des Weiteren hat sich herausgestellt, dass auch die Temperatur, bei der die $C_1$-$C_6$-Alkohole in Schritt (2) aus dem Reaktionsgemisch entfernt werden, einen wesentlichen Einflussfaktor im Hinblick auf die Leistungsfähigkeit des späteren Haarbehandlungsproduktes darstellen kann.

In diesem Zusammenhang wird vermutet, dass zu heiße Temperaturen oberhalb von 70 °C die Kondensation in Richtung hochmolekularer Produkte verschieben, die zu groß sind, um sich bei der späteren Keratinbehandlung als geschlossener und resistenter Film auf dem Keratinmaterial abzulagern. Aus diesem Grund wird ist es besonders bevorzugt, bei der Entfernung der $C_1$-$C_6$-Alkohole aus dem Reaktionsgemisch einen Temperaturbereich von 20 bis 70 °C einzuhalten.

Besonders bevorzugt ist es, bei der Entfernung der durch die Reaktion in Schritt (1) freigesetzten $C_1$-$C_6$-Alkohole aus dem Reaktionsgemisch einen Temperaturbereich von 20 °C bis 70 °C, bevorzugt 20 bis 65 °C, weiter bevorzugt von 20 bis 60 °C, noch weiter bevorzugt von 20 bis 55 °C, noch weiter bevorzugt von 20 bis 50 °C und ganz besonders bevorzugt von 20 bis 45 °C einzuhalten.

Auch in Schritt (2) bezieht sich der angegebene Temperaturbereich wieder auf die Temperatur, auf die das Reaktionsgemisch eingestellt sein muss, während die $C_1$-$C_6$-Alkoxy-Silane aus dem Reaktionsgemisch entfernt werden. Gemessen werden kann auch diese Temperatur beispielsweise durch ein in diese Mischung hineinragendes, geeichtes Thermometer.

In einer weiteren ganz besonders bevorzugten Ausführungsform ist ein erfindungsgemäßes Verfahren gekennzeichnet durch die

(2) partielle oder vollständige Entfernung der durch die Reaktion in Schritt (1) freigesetzten $C_1$-$C_6$-Alkohole aus dem Reaktionsgemisch bei einer Temperatur von 20 °C bis 70 °C, bevorzugt von 20 bis 65 °C, weiter bevorzugt von 20 bis 60 °C, noch weiter bevorzugt von 20 bis 55 °C, noch weiter bevorzugt von 20 bis 50 °C und ganz besonders bevorzugt von 20 bis 45 °C.

In Schritt (2) des Verfahrens kann die Einstellung der erfindungsgemäßen und bevorzugten Temperaturbereiche beispielsweise durch Erhitzen bzw, Kühlen des Reaktionsgefäßes oder Reaktors erfolgen, zum Beispiel durch Einsatz des Reaktionsgefäßes in einen Heizpilz, oder dadurch, dass das Reaktionsgefäß von außen mit einem temperierten Bad umgeben wird. Bei dem Temperierbad kann es sich beispielsweise um ein Wasserbad oder um Silikonölbad handeln.

Wird die Reaktion in einem Doppelwandreaktor durchgeführt, so kann auch eine temperierte Flüssigkeit durch den Raum geleitet werden, der durch die beiden Wandungen gebildet wird und der den Reaktionsraum umgibt.

Um in Schritt (2) des Verfahrens eine möglichst vollständige Entfernung der freigesetzten $C_1$-$C_6$-Alkohole ohne Überschreitung des bevorzugten Temperaturbereichs zu gewährleisten, werden die $C_1$-$C_6$-Alkohole bevorzugt unter (im Vergleich zum Normaldruck) reduziertem Druck entfernt. Als besonders vorteilhaft hat es sich in diesem Zusammenhang erwiesen die $C_1$-$C_6$-Alkohole mittels einem Destillationsaufsatz aus dem Reaktionsgemisch abzudestillieren. Bei dieser Destillation wird bevorzugt ein Druck von 10 bis 900 mbar, weiter bevorzugt von 10 bis 800 mbar, noch weiter bevorzugt von 10 bis 600 mbar und ganz besonders bevorzugt von 10 bis 300 mbar eingestellt.

Die Vakuumdestillation ist ein übliches chemisches Verfahren, für das die gängigen kommerziell erwerblichen Vakuumpumpen und Destillationsapparaturen eingesetzt werden können. Die Destillationsapparaturen können als Aufsatz auf dem Reaktionsgefäß oder Reaktor vorliegen.

In einer weiteren ganz besonders bevorzugten Ausführungsform ist ein erfindungsgemäßes Verfahren gekennzeichnet durch die

(2) partielle oder vollständige Entfernung der durch die Reaktion in Schritt (1) freigesetzten $C_1$-$C_6$-Alkohole aus dem Reaktionsgemisch mittels Destillation bei einem Druck von 10 bis 900 mbar, weiter bevorzugt von 10 bis 800 mbar, noch weiter bevorzugt von 10 bis 600 mbar und ganz besonders bevorzugt von 10 bis 300 mbar.

Eine weitere Möglichkeit, eine möglichst vollständige Entfernung der durch die Reaktion in Schritt (1) freigesetzten $C_1$-$C_6$-Alkohole zu gewährleisten, besteht darin, die Destillation über bestimmte Mindestzeiträume durchzuführen. Die Dauer der Destillation wird von der im erfindungsgemäßen Verfahren gewählten Ansatzgröße mitbestimmt. Bei einer üblichen Ansatzgröße von bis zu 50 kg, bevorzugt von bis zu 20 kg, kann es jedoch von ganz besonderem Vorteil sein, die Destillation, insbesondere unter Einstellung der vorgenannten Temperatur- und Druckbedingungen, über einen Zeitraum von mindestens 90 Minuten, bevorzugt mindestens 120 Minuten, weiter bevorzugt von mindestens 150 Minuten und ganz besonders bevorzugt bei mindestens 180 Minuten durchzuführen. Nach Ablauf von beispielsweise 300 Minuten ist die Destillation dann abgeschlossen.

In einer weiteren ganz besonders bevorzugten Ausführungsform ist ein erfindungsgemäßes Verfahren gekennzeichnet durch die

(2) partielle oder vollständige Entfernung der durch die Reaktion in Schritt (1) freigesetzten $C_1$-$C_6$-Alkohole aus dem Reaktionsgemisch mittels Destillation über einen Zeitraum von 90 bis 300 Minuten, bevorzugt 120 bis 300 Minuten, weiter bevorzugt 150 bis 300 Minuten und ganz besonders bevorzugt 180 bis 300 Minuten.

Im Anschluss an die Vakuumdestillation können die flüchtigen Alkohole und ggf. auch mit abdestilliertes Wasser kondensiert und als flüssiges Destillat in einer Vorlage aufgefangen werden. Die Destillation kann optional unter Kühlung der verdampften Alkohole/Wasser mittels eines Kühlers erfolgen. Der reduzierte Druck kann mittels üblicher und im Stand der Technik bekannter Verfahren erzeugt werden, typischerweise mit einer Vakuumpumpe.

Wie bereits beschrieben werden im erfindungsgemäßen Verfahren ganz besonders bevorzugt $C_1$-$C_6$-Alkoxysilane eingesetzt, die Methoxysilan- oder Ethoxysilangruppen tragen, insbesondere Diund Trimethoxy- und -ethoxysilane, besonders bevorzugt Trimethoxy- oder Triethoxysilane. Diese haben den Vorteil, dass bei der Hydrolyse und Kondensation Methanol bzw. Ethanol freigesetzt werden, die aufgrund ihrer Siedepunkte leicht mittels Vakuumdestillation aus der Reaktionsmischung entfernt werden können.

<u>Zugabe eines oder mehrerer kosmetischer Inhaltsstoffe in Schritt (3)</u>

Als optionalen Schritt (3) umfasst das erfindungsgemäße Verfahren die Zugabe eines oder mehrerer kosmetischer Inhaltsstoffe.

Entsprechend der Maßgabe, dass der Gesamtgehalt an $C_1$-$C_6$-Alkoholen in der nach Schritt (4) erhaltenen Zubereitung - bezogen auf das Gesamtgewicht der Zubereitung - unterhalb von 10,0 Gew.-% liegen muss, werden in Schritt (3) bevorzugt keine $C_1$-$C_6$-Alkohole zugegeben.

Insbesondere wird in Schritt (3) des Verfahrens kein $C_1$-$C_6$-Alkohol aus der Gruppe aus Methanol, Ethanol, n-Propanol, Isopropanol, 1-Butanol, 2-Butanol, 1-Pentanol, 2-Pentanol, 3-Pentanol, 1-Hexanol, 2-Hexanol, 3-Hexanol,

Ethylenglycol (1,2-Ethandiol), 1,2-Propandiol, 1,3-Propandiol und Glycerin zur Zubereitung hinzugegeben.

Bei den fakultativ in Schritt (3) einsatzbaren kosmetischen Inhaltsstoffen kann es sich um alle geeigneten Bestandteile handeln, um dem Mittel weitere positive Eigenschaften zu verleihen. Beispielsweise können in Schritt (3) des Verfahrens kosmetische Inhaltsstoffe aus der Gruppe der von $C_1$-$C_6$-Alkoholen verschiedenen Lösungsmittel, der verdickenden oder filmbildenden Polymere, der oberflächenaktiven Verbindungen aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der $C_8$-$C_{30}$-Fettalkohole, der Kohlenwasserstoffverbindungen, Fettsäureester, der zur Gruppe der pH-Regulatoren zugehörigen Säuren und Basen, der Parfüme, der Konservierungsmittel, der Pflanzenextrakte und der Proteinhydrolysate hinzugegeben werden.

In einer weiteren bevorzugten Ausführungsform ist ein erfindungsgemäßes Verfahren gekennzeichnet durch die (3) Zugabe eines oder mehrerer kosmetischer Inhaltsstoffe aus der Gruppe der von $C_1$-$C_6$-Alkoholen verschiedenen Lösungsmittel, der Polymere, der oberflächenaktiven Verbindungen, der farbgebenden Verbindungen, der Fettkomponenten, der pH-Regulatoren, der Parfüme, der Konservierungsmittel, der Pflanzenextrakte und der Proteinhydrolysate.

In einer weiteren ganz besonders bevorzugten Ausführungsform ist ein erfindungsgemäßes Verfahren gekennzeichnet durch die

(3) Zugabe eines oder mehrerer kosmetischer Inhaltsstoffe aus der Gruppe der Polymere, der oberflächenaktiven Verbindungen, der farbgebenden Verbindungen, der Fettkomponenten, der pH-Regulatoren, der Parfüme, der Konservierungsmittel, der Pflanzenextrakte und der Proteinhydrolysate.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

Als ganz besonders bevorzugt hat es sich in diesem Zusammenhang erwiesen, in Schritt (3) einen kosmetischen Inhaltsstoff einzusetzen, welcher die Stabilität, insbesondere die Lagerstabilität des Keratinbehandlungsmittels weiter verbessert. In diesem Zusammenhang hat sich die Zugabe (3) eines oder mehrerer kosmetischer Inhaltsstoffe aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan als besonders vorteilhaft im Hinblick auf die Erhöhung der Stabilität der Zusammensetzung erwiesen.

In einer weiteren ganz besonders bevorzugten Ausführungsform ist ein erfindungsgemäßes Verfahren gekennzeichnet durch die

(3) Zugabe eines oder mehrerer kosmetischer Inhaltsstoffe aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan.

Hexamethyldisiloxan besitzt die CAS-Nummer 107-46-0 und kann beispielsweise bei Sigma-Aldrich kommerziell erworben werden.

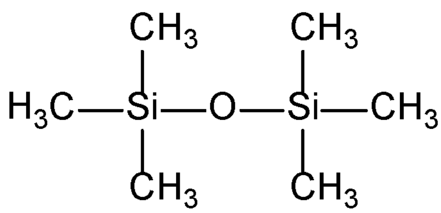

Octamethyltrisiloxan besitzt die CAS-Nummer 107-51-7 und ist ebenfalls bei Sigma-Aldrich kommerziell erhältlich.

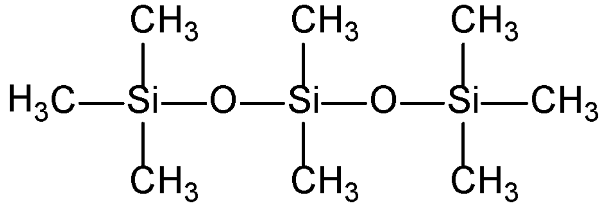

Decamethyltetrasiloxan trägt die CAS-Nummer 141-62-8 und ist ebenfalls bei Sigma-Aldrich kommerziell erhältlich.

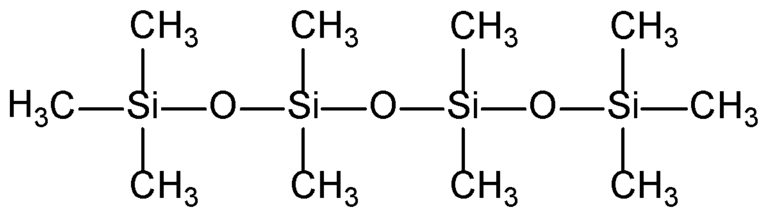

Hexamethylcyclotrisiloxan besitzt die CAS-Nr. 541-05-9.
Octamethylcyclotetrasiloxan besitzt die CAS-Nr. 556-67-2.
Decamethylcyclopentasiloxan besitzt die CAS-Nr. 541-02-6.

Abfüllung der Zubereitung in eine Verpackungseinheit (4)

In Schritt (4) des erfindungsgemäßen Verfahrens wird die nach den Schritten (1) und (2) - sowie gegebenenfalls nach dem optionalen Schritt (3) - erhaltene Zubereitung in eine Verpackungseinheit abgefüllt.

Bei der Verpackungseinheit kann es sich entweder um eine Endverpackung handeln, aus welcher der Anwender das Mittel zur Behandlung der Keratinmaterialien entnimmt. Geeignete Endverpackungen sind beispielsweise eine Flasche, eine Tube, einen Tiegel, eine Dose, ein Sachet, einen Aerosol-Druckbehälter, einen Non-Aerosol-Druckbehälter. Hierbei können diese Endverpackungen die Keratinbehandlungsmittel in Mengen enthalten, die für eine, gegebenenfalls auch führ mehrere Anwendungen ausreichen. Bevorzugt ist die Abfüllung in einer Menge, die für eine einmalige Anwendung ausreichend ist.

Weiterhin kann die Zubereitung in Schritt (4) jedoch auch in eine Zwischenverpackung abgefüllt werden, bei der es sich beispielsweise um einen Kanister oder einen Hobbock handeln kann. Die Abfüllung in eine Zwischenverpackung ist insbesondere dann geeignet, wenn das Reaktionsgefäß bwz. der Reaktor, in dem das erfindungsgemäße Verfahren durchgeführt wurde, und die Abfüllanlage, in der eine Abfüllung in die Endverpackung erfolgt, räumlich getrennt sind.

In einer weiteren ganz besonders bevorzugten Ausführungsform ist ein erfindungsgemäßes Verfahren gekennzeichnet durch die
(4) Abfüllung der Zubereitung in eine Flasche, eine Tube, einen Tiegel, eine Dose, ein Sachet, einen Aerosol-Druckbehälter, einen Non-Aerosol-Druckbehälter, einen Kanister oder einen Hobbock.

Bei den vorgenannten Verpackungseinheiten kann es sich um übliche, standardmäßig in der Kosmetik eingesetzte und kommerziell erhältliche Behältnisse handeln.

Gesamtgehalt an $C_1$-$C_6$-Alkoholen in der nach Schritt (4) erhaltenen Zubereitung

Bei der Anwendung der durch das erfindungsgemäße Verfahren hergestellten Zubereitung auf dem Keratinmaterial ist die Erzeugung eines stabilen, zusammenhängenden und gleichmäßigen Coatings die Grundvoraussetzung für die Erzielung der gewünschten anwendungstechnischen Eigenschaften. Intensive und langanhaltende Färbungen können vor allem dann erhalten werden, wenn die farbgebenden Verbindungen in ein entsprechend resistentes Coating integriert werden können. Wie zuvor geschrieben, ist es hierfür wesentlich, den Gehalt an $C_1$-$C_6$-Alkoholen in der final nach Schritt (4) erhaltenen Zubereitungen möglichst gering zu halten. Aus diesem Grund besteht die Maßgabe, dass der Gesamtgehalt an $C_1$-$C_6$-Alkoholen in der nach Schritt (4) erhaltenen Zubereitung - bezogen auf das Gesamtgewicht der Zubereitung - unterhalb von 10,0 Gew.-% liegt.

Bei der nach Schritt (4) erhaltenen Zubereitung, die durch ihren Maximalgehalt an $C_1$-$C_6$-Alkoholen bestimmt ist, handelt es sich um die Zubereitung, die nach ihrer Herstellung in eine Verpackungseinheit abgefüllt wurde. Hierbei erfolgt die Bestimmung bwz. Messung des Gehalts an $C_1$-$C_6$-Alkoholen erfindungsgemäß innerhalb von 24 Stunden nach der Abfüllung. Die Angabe des in der Zubereitung maximal enthaltenen Mengenanteils an $C_1$-$C_6$-Alkoholen in Gewichtsprozent bezieht sich auf das Gesamtgewicht der Zubereitung, wie sie nach der Abfüllung in der Verpackungseinheit nach Schritt (4) vorliegt.

Unter $C_1$-$C_6$-Alkoholen im Sinne der Erfindung werden Alkohole mit einer Hydroxygruppe oder mehreren Hydroxy-Gruppen verstanden, die 1 bis 6 Kohlenstoffatome umfassen. Diese Alkohole können linear oder verzweigt, gesättigt oder einfach bzw. mehrfach ungesättigt sein. Unter $C_1$-$C_6$-Mono-Alkoholen werden insbesondere die Alkohole aus der Gruppe aus Methanol, Ethanol, n-Propanol, Isopropanol, 1-Butanol, 2-Butanol, 1-Pentanol, 2-Pentanol, 3-Pentanol, 1-Hexanol, 2-Hexanol und 3-Hexanol verstanden. $C_1$-$C_6$-Alkohole mit zwei Hydroxygruppen sind beispielsweise Ethylenglycol, 1,2-Propandiol und 1,3-Propandiol. Ein $C_1$-$C_6$-Alkohol mit drei Hydroxygruppen ist beispielsweise Glycerin.

Zur Einhaltung dieser Gewichtsvorgaben müssen einerseits die $C_1$-$C_6$-Alkohole in Schritt (2) möglichst vollständig aus der Reaktionsgemisch entfern werden, andererseits dürfen in Schritt (3) auch keine entsprechenden $C_1$-$C_6$-Alkohole zur Zubereitung hinzugegeben werden.

Mit Zubereitungen, deren Gesamtgehalt an $C_1$-$C_6$-Alkoholen bei 10,0 Gew.-% lag, konnten bei Anwendung auf dem Keratinmaterial Färbungen mit ausreichend hoher Farbintensität erhalten werden.

Noch bessere Ergebnisse wurden jedoch erhalten, wenn der Gesamtgehalt an $C_1$-$C_6$-Alkoholen in der nach Schritt (4) erhaltenen Zubereitung - bezogen auf das Gesamtgewicht der Zubereitung - auf einen Wert unterhalb von 9,0 Gew.-%, bevorzugt unterhalb von 8,0 Gew.-%, weiter bevorzugt unterhalb von 7,0 Gew.-%, noch weiter bevorzugt unterhalb von 6,0 Gew.-% und ganz besonders bevorzugt unterhalb von 5,0 Gew.-% reduziert werden konnte.

In einer weiteren ganz besonders bevorzugten Ausführungsform ist ein erfindungsgemäßes Verfahren dadurch gekennzeichnet, dass der Gesamtgehalt an $C_1$-$C_6$-Alkoholen in der nach Schritt (4) erhaltenen Zubereitung - bezogen auf das Gesamtgewicht der Zubereitung - unterhalb von 9,0 Gew.-%, bevorzugt unterhalb von 8,0 Gew.-%, weiter bevorzugt unterhalb von 7,0 Gew.-%, noch weiter bevorzugt unterhalb von 6,0 Gew.-% und ganz besonders bevorzugt unterhalb von 5,0 Gew.- % liegt.

In einer weiteren ganz besonders bevorzugten Ausführungsform ist ein erfindungsgemäßes Verfahren dadurch gekennzeichnet, dass der Gesamtgehalt an $C_1$-$C_6$-Alkoholen in der nach Schritt (4) erhaltenen Zubereitung - bezogen auf das Gesamtgewicht der Zubereitung - unterhalb von 10,0 Gew.-%, bevorzugt unterhalb von 9,0 Gew.-%, weiter bevorzugt unterhalb von 8,0 Gew.-%, noch weiter bevorzugt unterhalb von 7,0 Gew.-%, noch weiter bevorzugt unterhalb von 6,0 Gew.-%, und ganz besonders bevorzugt unterhalb von 5,0 Gew.-% liegt, wobei die Bestimmung des Gehaltes an $C_1$-$C_6$-Alkoholen innerhalb von 24 Stunden nach der Abfüllung der Zubereitung in eine Verpackungseinheit (4) erfolgt.

Mit anderen Worten ist ein bevorzugtes erfindungsgemäßes Verfahren dadurch gekennzeichnet, dass der Gesamtgehalt an $C_1$-$C_6$-Alkoholen in der Zubereitung innerhalb von 24 Stunden nach der Abfüllung in eine Verpackungseinheit (4) - bezogen auf das Gesamtgewicht der Zubereitung - unterhalb von 10,0 Gew.-%, bevorzugt unterhalb von 9,0 Gew.-%, weiter bevorzugt unterhalb von 8,0 Gew.-%, noch weiter bevorzugt unterhalb von 7,0 Gew.-%, noch weiter bevorzugt unterhalb von 6,0 Gew.-%, und ganz besonders bevorzugt unterhalb von 5,0 Gew.-% liegt.

Die Bestimmung des Gehalts an $C_1$-$C_6$-Alkoholen in der nach Schritt (4) erhaltenen Zubereitung kann mittels verschiedener analytischer Methoden erfolgen. Eine Möglichkeit ist die Messung mittels GC-MS. Die Gaschromatographie mit Massenspektrometrie-Kopplung ist die Kopplung eines Gas-Chromatographen (GC) mit einem Massenspektrometer (MS). Das Gesamtverfahren bzw. die Gerätekoppelung wird verkürzend auch als GC-MS, GC/MS oder GCMS bezeichnet

Zur Bestimmung des Gehaltes an $C_1$-$C_6$-Alkoholen kann eine Probe der Zubereitung beispielsweise in einer Doppelbestimmung auf einer unpolaren Säule gaschromatographisch untersucht werden. Die Identifizierung der zugeordneten Komponenten kann massenspektrometrisch über Bibliotheksvergleichsspektren (z.B. NIST oder Wiley) erfolgen. Aus den Doppelbestimmungen wird jeweils der Mittelwert gebildet. Quantifiziert werden kann beispielsweise mittels interner Standard-Kalibrierung (z.B. mit Methylisobutylketon).

Wassergehalt der nach Schritt (4) erhaltenen Zubereitung

Um eine möglichst lagerstabile Formulierung bereitstellen zu können, besitzt die nach Schritt (4) erhaltene Zubereitung bevorzugt einen möglichst geringen Wassergehalt. Der Wassergehalt des Produkts nach der Vakuumdestillation liegt daher bevorzugt unterhalb von 5,0 Gew.-%, bevorzugt unterhalb von 4,0 Gew.-%, weiter bevorzugt unterhalb von 3,0 Gew.-%, noch weiter bevorzugt unterhalb von 2,0 Gew.-% und ganz besonders bevorzugt unterhalb von 1,0 Gew.-%. Hierbei wird der in Gew.-% angegebene Wassergehalt der Zubereitung zum Gesamtgewicht der nach Schritt (4) erhaltenen Zubereitung in Relation gesetzt.

In einer weiteren ganz besonders bevorzugten Ausführungsform ist ein erfindungsgemäßes Verfahren dadurch gekennzeichnet, dass der Wassergehalt in der nach Schritt (4) erhaltenen Zubereitung - bezogen auf das Gesamtgewicht der Zubereitung - unterhalb von 5,0 Gew.-%, bevorzugt unterhalb von 4,0 Gew.-%, weiter bevorzugt unterhalb von 3,0 Gew.-%, noch weiter bevorzugt unterhalb von 2,0 Gew.-% und ganz besonders bevorzugt unterhalb von 1,0 Gew.-% liegt.

Mit anderen Worten ist ein bevorzugtes erfindungsgemäßes Verfahren dadurch gekennzeichnet, dass der Wassergehalt in der nach Schritt (4) erhaltenen Zubereitung - bezogen auf das Gesamtgewicht der Zubereitung - unterhalb von 5,0 Gew.-%, bevorzugt unterhalb von 4,0 Gew.-%, weiter bevorzugt unterhalb von 3,0 Gew.-%, noch weiter bevorzugt unterhalb von 2,0 Gew.-% und ganz besonders bevorzugt unterhalb von 1,0 Gew.-% liegt, wobei die Bestimmung des Wassergehaltes innerhalb von 24 Stunden nach der Abfüllung der Zubereitung in eine Verpackungseinheit (4) erfolgt.

Bevorzugt ist ein Verfahren zur Herstellung eines Mittels zur Behandlung von keratinischem Material, insbesondere menschlichen Haaren, umfassend die folgenden Schritte:

(1) Mischen eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem

Reaktionsgemisch,
(3) gegebenenfalls Zugabe eines oder mehrerer kosmetischer Inhaltsstoffe, und
(4) Abfüllung der Zubereitung in eine Verpackungseinheit,
dadurch gekennzeichnet, dass

- der Gesamtgehalt an $C_1$-$C_6$-Alkoholen in der nach Schritt (4) erhaltenen Zubereitung - bezogen auf das Gesamtgewicht der Zubereitung - unterhalb von 10,0 Gew.-% liegt, und
- der Wassergehalt in der nach Schritt (4) erhaltenen Zubereitung - bezogen auf das Gesamtgewicht der Zubereitung - unterhalb von 5,0 Gew.-% liegt.

Bevorzugt ist ein Verfahren zur Herstellung eines Mittels zur Behandlung von keratinischem Material, insbesondere menschlichen Haaren, umfassend die folgenden Schritte:

(1) Mischen eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,
dadurch gekennzeichnet, dass

- der Gesamtgehalt an $C_1$-$C_6$-Alkoholen in der nach Schritt (4) erhaltenen Zubereitung - bezogen auf das Gesamtgewicht der Zubereitung - unterhalb von 9,0 Gew.-% liegt, und
- der Wassergehalt in der nach Schritt (4) erhaltenen Zubereitung - bezogen auf das Gesamtgewicht der Zubereitung - unterhalb von 4,0 Gew.-% liegt.

Bevorzugt ist ein Verfahren zur Herstellung eines Mittels zur Behandlung von keratinischem Material, insbesondere menschlichen Haaren, umfassend die folgenden Schritte:

(1) Mischen eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,
dadurch gekennzeichnet, dass

- der Gesamtgehalt an $C_1$-$C_6$-Alkoholen in der nach Schritt (4) erhaltenen Zubereitung - bezogen auf das Gesamtgewicht der Zubereitung - unterhalb von 8,0 Gew.-% liegt, und
- der Wassergehalt in der nach Schritt (4) erhaltenen Zubereitung - bezogen auf das Gesamtgewicht der Zubereitung - unterhalb von 3,0 Gew.-% liegt.

Bevorzugt ist ein Verfahren zur Herstellung eines Mittels zur Behandlung von keratinischem Material, insbesondere menschlichen Haaren, umfassend die folgenden Schritte:

(1) Mischen eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,
dadurch gekennzeichnet, dass

- der Gesamtgehalt an $C_1$-$C_6$-Alkoholen in der nach Schritt (4) erhaltenen Zubereitung - bezogen auf das Gesamtgewicht der Zubereitung - unterhalb von 7,0 Gew.-% liegt, und
- der Wassergehalt in der nach Schritt (4) erhaltenen Zubereitung - bezogen auf das Gesamtgewicht der Zubereitung - unterhalb von 2,0 Gew.-% liegt.

Bevorzugt ist ein Verfahren zur Herstellung eines Mittels zur Behandlung von keratinischem Material, insbesondere menschlichen Haaren, umfassend die folgenden Schritte:

(1) Mischen eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,
dadurch gekennzeichnet, dass

- der Gesamtgehalt an $C_1$-$C_6$-Alkoholen in der nach Schritt (4) erhaltenen Zubereitung - bezogen auf das Gesamtgewicht der Zubereitung - unterhalb von 6,0 Gew.-% liegt, und
- der Wassergehalt in der nach Schritt (4) erhaltenen Zubereitung - bezogen auf das Gesamtgewicht der Zubereitung - unterhalb von 1,0 Gew.-% liegt.

Abfolge der Verfahrensschritte

Es ist kennzeichnend für das erfindungsgemäße Verfahren, dass es die Schritte (1), (2), (3) und (4) umfasst, wobei der Schritt (3) ein optionaler Schritte sind. Betreffend die Abfolge der Verfahrensschritte sind mehrere Ausführungsformen geeignet.

Im Rahmen einer Ausführungsform bevorzugt ist ein Verfahren umfassend die Schritte in der folgenden Reihenfolge:

(1) Reaktion eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,

Dieses Verfahren beginnt mit Schritt (1), gefolgt von Schritt (2), gefolgt von Schritt (3), gefolgt von Schritt (4). Zunächst werden ein oder mehrere organische $C_1$-$C_6$-Alkoxy-Silane mit Wasser vermischt, und die bei dieser Reaktion entstehen $C_1$-$C_6$-Alkohole werden in Schritt (2) so vollständig wie möglich entfernt. Danach werden dem Reaktionsgemisch ein oder mehrere kosmetische Inhaltsstoffe hinzugefügt, die beispielsweise ein aprotisches Lösungsmittel, ein Pigment, ein verdickendes Polymer o.ä. sein können (Schritt 3). Danach wird die Zubereitung in eine Verpackungseinheit abgefüllt (Schritt 4).

Im Rahmen einer weiteren Ausführungsform kann es genauso bevorzugt sein, die Zugabe des bzw. der kosmetischen Inhaltsstoffe (3) vor Entfernung der $C_1$-$C_6$-Alkohole in Schritt (2) vorzunehmen.

Im Rahmen einer weiteren Ausführungsform bevorzugt ist ein Verfahren umfassend die Schritte in der folgenden Reihenfolge:

(1) Reaktion eines oder mehrerer organischer $C_1$-$C_6$-Alkoxy-Silane mit Wasser,
(3) gegebenenfalls Zugabe eines oder mehrerer kosmetischer Inhaltsstoffe,
(2) partielle oder vollständige Entfernung der durch die Reaktion in Schritt (1) freigesetzten $C_1$-$C_6$-Alkohole aus dem Reaktionsgemisch, und
(4) Abfüllung der Zubereitung in eine Verpackungseinheit.

pH-Werte der Zubereitungen im Verfahren

In weiteren Versuchen hat sich herausgestellt, dass auch die pH-Werte, die das Reaktionsgemisch im Verlauf der Schritte (1) bis (4) des erfindungsgemäßen Verfahrens besitzt, einen Einfluss auf die Kondensationsreaktion nehmen kann. Hierbei wurde gefunden, dass insbesondere alkalische pH-Werte die Kondensation auf der Stufe der Oligomere anhalten. Je saurer das Reaktionsgemisch ist, desto scheint die Kondensation stattzufinden und desto höher ist das Molekulargewicht der bei der Kondensation entstehenden Siloxane. Aus diesem Grund ist es bevorzugt, dass das Reaktionsgemisch in Schritt (1), (2), (3) und/oder (4) einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzt.

In einer weiteren ganz besonders bevorzugten Ausführungsform ist ein erfindungsgemäßes Verfahren, dadurch gekennzeichnet, dass das Reaktionsgemisch in Schritt (1), (2), (3) und/oder (4) nach Vermischen im Gewichtsverhältnis 1:1 mit Wasser einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzt.

In einer weiteren ganz besonders bevorzugten Ausführungsform ist ein erfindungsgemäßes Verfahren, dadurch gekennzeichnet, dass das Reaktionsgemisch in den Schritten (1) bis (6) nach Vermischen im Gewichtsverhältnis 1:1

mit Wasser einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzt.

Zur Einstellung dieses alkalischen pH-Wertes kann es erforderlich werden, der Reaktionsmischung ein Alkalisierungsmittel und/oder Acidifizierungsmittel zuzusetzen. Bei den pH-Werten im Sinne der vorliegenden Erfindung handelt es sich um pH-Werte, die bei einer Temperatur von 22°C gemessen wurden.

Als Alkalisierungsmittel können beispielsweise Ammoniak, Alkanolamine und/oder basische Aminosäuren eingesetzt werden.

Alkanolamine können ausgewählt werden aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol.

Als Aminosäure im Sinne der Erfindung gilt eine organische Verbindung, die in ihrer Struktur mindestens eine protonierbare Aminogruppe und mindestens eine -COOH- oder eine -SOsH-Gruppe enthält. Bevorzugte Aminosäuren sind Aminocarbonsäuren, insbesondere $\alpha$,-(alpha)-Amino-carbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können auch anorganische Alkalisierungsmittel eingesetzt werden. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierunsmitteln sind dem Fachmann zur Feineinstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Mittel zur Behandlung von keratinischem Material

Das zuvor beschriebene Verfahren erlaubt die Herstellung von vorhydrolysierten bzw. kondensierten Silan-Blends, die bei Anwendung auf keratinischem Material eine außerordentlich gute Leistung zeigen.

Prinzipiell können die mittels dieses Verfahrens hergestellten Keratin-Behandlungsmittel zu verschiedenen Zwecken eingesetzten werden, beispielsweise als Mittel zur Färbung von keratinischem Material, als Mittel zur Pflege von keratinischem Material oder als Mittel zur Formveränderung von keratinischem Material.

In einer weiteren ganz besonders bevorzugten Ausführungsform ist ein erfindungsgemäßes Verfahren dadurch gekennzeichnet, dass ein Mittel zur Färbung von keratinischem Material, zur Pflege von keratinischem Material oder zur Formveränderung von keratinischem Material hergestellt, gelagert und später angewendet wird.

In einer weiteren ganz besonders bevorzugten Ausführungsform ist ein erfindungsgemäßes Verfahren dadurch gekennzeichnet, dass ein Mittel zur Färbung von keratinischem Material, zur Pflege von keratinischem Material oder zur Formveränderung von keratinischem Material hergestellt wird.

Explizit ganz besonders gute Eignung zeigen die hergestellte Mittel bei Anwendung in einem Färbeverfahren.

In einer weiteren ganz besonders bevorzugten Ausführungsform ist ein erfindungsgemäßes Verfahren dadurch gekennzeichnet, dass ein Mittel zur Färbung von keratinischem Material hergestellt wird.

Bei Anwendung in einem Färbeverfahren kann dem Mittel beispielsweise in Schritt (3) mindestens eine farbgebende Verbindung zugesetzt werden, wobei die farbgebende Verbindung ausgewählt sein kann aus der Gruppe der

Pigmente, der direktziehenden Farbstoffe und/oder der Oxidationsfarbstoffvorprodukte. Hierbei kann ein Mittel zur Färbung von Keratinmaterial erhalten werden, welches neben den vorhydrolysierten/kondensierten C1-C6-Alkoxysilanen zusätzlich auch noch die farbgebende(n) Verbindung(en) enthält.

Mittel in Verpackungseinheit

Im Rahmen einer Ausführungsform können über das erfindungsgemäße Verfahren hergestellten Zubereitungen bei ihrer Anwendung direkt aus der Verpackungseinheit entnommen und vom Anwender auf dem Keratinmaterial appliziert werden.

Ein zweiter Gegenstand der Erfindung ist ein Mittel zur Behandlung von keratinischem Material, umfassend eine Zubereitung in einer Verpackungseinheit, die nach einem Verfahren hergestellt wurde, wie es bei der Beschreibung des ersten Erfindungsgegenstands im Detail offenbart wurde.

Mehrkomponenten-Verpackungseinheit (Kit-of-parts)

Im Rahmen einer weiteren Ausführungsform ist es jedoch ebenfalls ganz besonders bevorzugt, wenn die nach Schritt (4) des Verfahrens hergestellte Zubereitung zunächst mit einer weiteren Zubereitung vermischt wird, so dass ein anwendungsbereites Färbemittel erhalten wird. Dieses anwendungsbereite Färbemittel wird dann auf die Keratinmaterialien appliziert. Diese Ausführungsform ist insbesondere bei Anwendung der Zubereitungen in einem Färbeverfahren bevorzugt.

Zur Erhöhung des Anwender-Komforts werden dem Anwender alle für den Färbeprozess notwendigen Zubereitungen in Form einer Mehrkomponenten-Verpackungseinheit (Kit-of-parts) zur Verfügung gestellt.

Ein dritter Gegenstand der Erfindung ist eine Mehrkomponenten-Verpackungseinheit (Kit-of-parts) zum Färben von keratinischem Material, insbesondere menschlichen Haaren, welche getrennt voneinander konfektioniert

- eine erste Verpackungseinheit enthaltend eine kosmetische Zubereitung (A) und
- eine zweite Verpackungseinheit enthaltend eine kosmetische Zubereitung (B) umfasst, wobei
- die kosmetische Zubereitung (A) in der ersten Verpackungseinheit nach dem Verfahren hergestellt wurde, wie es bei der Beschreibung des ersten Erfindungsgegenstands im Detail offenbart wurde, und
- die kosmetische Zubereitung (B) mindestens eine farbgebende Verbindung aus der Gruppe der Pigmente, der direktziehenden Farbstoffe und/oder der Oxidationsfarbstoffvorprodukte enthält.

Kurz vor der Anwendung werden dann die beiden Zubereitungen (A) und (B) miteinander vermischt, und dieses anwendungsbereite Färbemittel wird dann auf das Keratinmaterial appliziert.

Weiterhin kann die erfindungsgemäße Mehrkomponenten-Verpackungseinheit auch noch eine dritte Verpackungseinheit enthaltend eine kosmetische Zubereitung (C) umfassen. Bei der Zubereitung (C) kann es sich beispielsweise um einen Conditioner, ein Shampoo, oder ein Vor- oder Nachbehandlungsmittel handeln.

Farbgebende Verbindungen

Bei Anwendung der über das erfindungsgemäße Verfahren hergestellten Mittel in einem Färbeverfahren können eine oder mehrere farbgebende Verbindungen zum Einsatz kommen. Die farbgebenden Verbindung(en) können entweder in Schritt (3) des Verfahrens als kosmetische Inhaltstoffe zum dem Reaktionsgemisch hinzugegeben werden, oder aber dem Anwender als Inhaltsstoff einer separat konfektionierten Zubereitung (B) zur Verfügung gestellt werden.

Das oder die farbgebenden Verbindungen können vorzugsweise ausgewählt werden aus der Pigmente, der direktziehenden Farbstoffe, der Oxidationsfarbstoffe, der photochromen Farbstoffe und der thermochromen Farbstoffe, insbesondere bevorzugt aus Pigmenten und/oder direktziehenden Farbstoffen.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist en erfindungsgemäßes Mittel dadurch gekennzeichnet, dass es mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte farbgebende Verbindungen aus der Gruppe der Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es (b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbgebenden Verbindungen auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (b) mindestens eine farbgebende Verbindung enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)

Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel bzw. die erfindungsgemäße Zubereitung auch ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einerweiteren besonders bevorzugten Ausführungsform ist ein erfindungsgemäßes Mittel dadurch gekennzeichnet, dass es mindestens eine farbgebende Verbindungen aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße $D_{50}$ von 1,0 bis 50 μm, vorzugsweise von 5,0 bis 45 μm, bevorzugt von 10 bis 40 μm, insbesondere von 14 bis 30 μm, aufweist. Die mittlere Teilchengröße $D_{50}$ kann beispielsweise unter Verwendung von dynamischer Lichtstreuung (DLS) bestimmt werden.

Das oder die Pigmente können in einer Menge von 0,001 bis 20 Gew.-%, insbesondere von 0,05 bis 5 Gew.-%, jeweils bezogen auf das Gesamtgewicht des erfindungsgemäßen Mittels bzw. der Zubereitung, eingesetzt werden.

Als farbgebende Verbindungen können die erfindungsgemäßen Mittel auch einen oder mehrere direktziehende Farbstoffe enthalten. Bei direktziehende Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L. Besonders bevorzugt besitzen die direktziehenden Farbstoffe im Sinne der vorliegenden Erfindung eine Löslichkeit in Wasser (760 mmHg) bei 25 °C von mehr als 1,5 g/L.

Direktziehende Farbstoffe können in anionische, kationische und nichtionische direktziehende Farbstoffe unterteilt werden.

In einer weiteren bevorzugten Ausführungsform ist ein erfindungsgemäßes Mittel dadurch gekennzeichnet, dass es als farbgebende Verbindung mindestens einen anionischen, kationischen und/oder nichtionischen direktziehenden Farbstoff enthält.

In einer weiteren bevorzugten Ausführungsform ist ein erfindungsgemäßes Mittel dadurch gekennzeichnet, dass es mindestens einen anionischen, kationischen und/oder nichtionischen direktziehenden Farbstoff enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung ($-SO_3H$) besitzen. In Abhängigkeit vom pH-Wert liegen die protonierten Formen (-COOH, $-SO_3H$) der Carbonsäure- bzw. Sulfonsäuregruppierungen mit ihren deprotonierten Formen ($-COO^-$, $-SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen,

Rhodamin-Farbstoffen, Oxazinfarbstoffen und/oder Indophenolfarbstoffen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201 ;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%. Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Weiterhin können auch thermochrome Farbstoffe eingesetzt werden. Thermochromie beinhaltet die Eigenschaft eines Materials, in Abhängigkeit der Temperatur reversibel oder irreversibel seine Farbe zu ändern. Dies kann sowohl durch Änderung der Intensität und/oder des Wellenlängenmaximums erfolgen.

Schließlich ist es auch möglich, photochrome Farbstoffe einzusetzen. Photochromie beinhaltet die Eigenschaft eines Materials, in Abhängigkeit der Bestrahlung mit Licht, insbesondere UV-Licht, reversibel oder irreversibel seine

Farbe zu ändern. Dies kann sowohl durch Änderung der Intensität und/oder des Wellenlängenmaximums erfolgen.

Auch die Zubereitung (B) kann zusätzlich noch einen oder mehrere weitere Inhaltsstoffe enthalten, die aus der Gruppe der Lösungsmittel, verdickenden oder filmbildenden Polymere, der oberflächenaktiven Verbindungen aus der Gruppe der nichtionischen, kationischen, anionischen oder zwitterionischen/amphoteren Tenside, der Fettkomponenten aus der Gruppe der $C_8$-$C_{30}$-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate ausgewählt sein können.

Betreffend die weiteren bevorzugten Ausführungsformen der erfindungsgemäßen Mittel und der Mehrkomponenten-Verpackungseinheit gilt *mutatis mutantis* das zum erfindungsgemäßen Verfahren gesagte.

Beispiele

1. Herstellung der Silan-Blends

1.1. Herstellung des Silan-Blends (Silan-Blend 1, Vergleich)

Ein Reaktor mit beheizbarer/kühlbarer Außenhülle und mit einem Fassungsvermögen von 10 Litern wurde mit 4,67 kg Methyltrimethoxysilan befüllt. Unter Rühren wurden dann 1,33 kg (3-Amino-propyl)triethoxysilan hinzugegeben. Dieses Gemisch wurde bei 30 °C gerührt. Im Anschluss daran wurden 670 ml Wasser (dest.) tropfenweise unter kräftigem Rühren hinzugegeben, wobei die Temperatur des Reaktionsgemisches unter externer Kühlung bei 30 °C gehalten wurde. Nach Beendigung der Wasserzugabe wurde für weitere 10 Minuten nachgerührt. Danach wurde ein Vakuum von 700 mbar angelegt und das Reaktionsgemisch auf eine Temperatur von 44 °C erwärmt. Sobald das Reaktionsgemisch die Temperatur von 44 °C erreicht hatte, wurden das bei der Reaktion freigesetzte Ethanol und Methanol über einen Zeitraum von 40 Minuten abdestilliert.

Die abdestillierten Alkohole wurden in einer gekühlten Vorlage aufgefangen. Danach ließ man das Reaktionsgemisch auf Raumtemperatur abkühlen. Zu dem auf diese Weise erhaltenen Gemisch wurden dann unter Rühren 3,33 kg Hexamethyldisiloxan getropft. Es wurde 10 Minuten nachgerührt. Jeweils 100 ml des Silan-Blends wurden in eine Flasche mit 100 ml Fassungsvermögen und Schraubdeckelverschluss mit Dichtung abgefüllt. Nach dem Abfüllen wurden die Flaschen fest verschlossen.

1.2. Herstellung des Silan-Blends (Silan-Blend 2, Erfindung)

Ein Reaktor mit beheizbarer/kühlbarer Außenhülle und mit einem Fassungsvermögen von 10 Litern wurde mit 4,67 kg Methyltrimethoxysilan befüllt. Unter Rühren wurden dann 1,33 kg (3-Amino-propyl)triethoxysilan hinzugegeben. Dieses Gemisch wurde bei 30 °C gerührt. Im Anschluss daran wurden 670 ml Wasser (dest.) tropfenweise unter kräftigem Rühren hinzugegeben, wobei die Temperatur des Reaktionsgemisches unter externer Kühlung bei 30 °C gehalten wurde. Nach Beendigung der Wasserzugabe wurde für weitere 10 Minuten nachgerührt. Danach wurde ein Vakuum von 280 mbar angelegt und das Reaktionsgemisch auf eine Temperatur von 44 °C erwärmt. Sobald das Reaktionsgemisch die Temperatur von 44 °C erreicht hatte, wurden das bei der Reaktion freigesetzte Ethanol und Methanol über einen Zeitraum von 190 Minuten abdestilliert.

Im Verlauf der Destillation wurde das Vakuum auf 200 mbar abgesenkt. Die abdestillierten Alkohole wurden in einer gekühlten Vorlage aufgefangen. Danach ließ man das Reaktionsgemisch auf Raumtemperatur abkühlen. Zu dem auf diese Weise erhaltenen Gemisch wurden dann unter Rühren 3,33 kg Hexamethyldisiloxan getropft. Es wurde 10 Minuten nachgerührt. Jeweils 100 ml des Silan-Blends wurden in eine Flasche mit 100 ml Fassungsvermögen und Schraubdeckelverschluss mit Dichtung abgefüllt. Nach dem Abfüllen wurden die Flaschen fest verschlossen.

2. Messung des Gehaltes an $C_1$-$C_6$-Alkoholen mittels GC-MS

Innerhalb von 24 Stunden nach ihrer Abfüllung wurde von beiden Silan-Blends der Gehalt an Ethanol und an Methanol mittels GC-MS Spektroskopie gemessen.

In einer Doppelbestimmung wurde jeweils eine Probe des Silan-Blends auf einer unpolaren Säule gaschromatographisch untersucht. Die Identifizierung der zugeordneten Komponenten erfolgte massenspektrometrisch über Bibliotheksvergleichsspektren (NIST /Wiley). Die Quantifizierung erfolgte mittels interner Standard-Kalibrierung mit Methylisobutylketon. Aus jeder Doppelbestimmung wurde jeweils der Mittelwert gebildet.

| | Silan-Blend 1 (Vergleich) | Silan-Blend 2 (Erfindung) |
|---|---|---|
| Methanol (Gew.-%) | 10,6 / 11,6 | 2,9 / 2,5 |

(fortgesetzt)

| | Silan-Blend 1 (Vergleich) | Silan-Blend 2 (Erfindung) |
|---|---|---|
| Ethanol (Gew.-%) | 4,6 / 5,3 | 1,5 / 1,4 |
| Summe Gehalt $C_1$-$C_6$-Alkohole (Mittelwert) | 16,1 | 4,2 |

3. Färbung

Es wurde das folgende Färbemittel bereitgestellt (Zubereitung (B)).

Zubereitung (B)

| | |
|---|---|
| Colorona Bordeaux, Merck, MICA, CI 77491 (IRON OXIDES) | 5,5 g |
| Hydroxyethylcellulose (Natrosol 250 HR) | 1,0 g |
| PEG-12 Dimethicone (Xiameter OFX-0193) | 2,0 g |
| Wasser | Ad 100 g |

Aus den zuvor hergestellten Flaschen mit Silan-Blend wurden jeweils 20 g abgewogen (Zubereitung A). Das anwendungsbereite Färbemittel wurde jeweils durch Verschütteln von 20 g der Zubereitung (A) und 100 g der Zubereitung (B) hergestellt (Schütteln für 3 Minuten). Dieses Gemisch wurde dann für 5 Minuten stehen gelassen.

Für die Anwendung wurde jeweils eine Haarsträhne (Kerling Euronaturhaar weiß) in das anwendungsbereite Färbemittel getaucht und für 1 Minute darin belassen. Danach wurde überflüssiges Mittel von jeder Haarsträhne gestreift. Dann wurde jede Haarsträhne mit Wasser ausgewaschen und getrocknet. Im Anschluss daran wurden die Strähnen visuell unter einer Tageslichtlampe bewertet. Hierbei wurden die folgenden Ergebnisse erhalten:

| Silan-Blend 1<br>Vergleich<br>20 g | Silan-Blend 2<br>Erfindung<br>20 g |
|---|---|

| Färbemittel (B)<br>100 g | Färbemittel (B)<br>100 g |
|---|---|
| Färbung: bordeaux-rot | Färbung: bordeaux-rot |
| Farbintensität: niedrig | Farbintensität: hoch |
| Deckvermögen: mittel | Deckvermögen: mittel |

**Patentansprüche**

1. Verfahren zur Herstellung eines Mittels zur Behandlung von keratinischem Material, insbesondere menschlichen Haaren, umfassend die folgenden Schritte:

   (1) Mischen eines oder mehrerer organischer $C_1$-$C_6$-Alkoxy-Silane mit Wasser,
   (2) partielle oder vollständige Entfernung der durch die Reaktion in Schritt (1) freigesetzten $C_1$-$C_6$-Alkohole aus dem Reaktionsgemisch,
   (3) gegebenenfalls Zugabe eines oder mehrerer kosmetischer Inhaltsstoffe, und
   (4) Abfüllung der Zubereitung in eine Verpackungseinheit,

   **dadurch gekennzeichnet, dass** der Gesamtgehalt an $C_1$-$C_6$-Alkoholen in der nach Schritt (4) erhaltenen Zubereitung - bezogen auf das Gesamtgewicht der Zubereitung - unterhalb von 10,0 Gew.-% liegt.

**2.** Verfahren nach Anspruch 1, **gekennzeichnet durch** das

(1) Mischen eines oder mehrerer organischer $C_1$-$C_6$-Alkoxy-Silane der Formel (I) und/oder (II) mit Wasser,

$$R_1R_2N\text{-}L\text{-}Si(OR_3)_a(R_4)_b \qquad (I)$$

wobei

- $R_1$, $R_2$ unabhängig voneinander für ein Wasserstoffatom oder eine $C_1$-$C_6$-Alkylgruppe stehen,
- L für eine lineare oder verzweigte, zweiwertige $C_1$-$C_{20}$-Alkylengruppe steht,
- $R_3$, $R_4$ unabhängig voneinander für eine $C_1$-$C_6$-Alkylgruppe stehen,
- a, für eine ganze Zahl von 1 bis 3 steht, und
- b für die ganze Zahl 3 - a steht, und

$$(R_5O)_c(R_6)_dSi\text{-}(A)_e\text{-}[NR_7\text{-}(A')]_f\text{-}[O\text{-}(A'')]_g\text{-}[NR_8\text{-}(A''')]_h\text{-}Si(R_6')_{d'}(OR_5')_{c'} \qquad (II),$$

wobei
- R5, R5', R5", R6, R6' und R6" unabhängig voneinander für eine $C_1$-$C_6$-Alkylgruppe stehen,
- A, A', A", A‴ und A⁗ unabhängig voneinander für eine lineare oder verzweigte, zweiwertige $C_1$-$C_{20}$-Alkylengruppe stehen,
- $R_7$ und $R_8$ unabhängig voneinander für ein Wasserstoffatom, eine $C_1$-$C_6$-Alkylgruppe, eine Hydroxy-$C_1$-$C_6$-alkylgruppe, eine $C_2$-$C_6$-Alkenylgruppe, eine Amino-$C_1$-$C_6$-alkylgruppe oder eine Gruppierung der Formel (III) stehen,
-

$$(A'''')\text{-}Si(R_6'')_{d''}(OR_5'')_{c''} \qquad (III),$$

- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

**3.** Verfahren nach einem der Ansprüche 1 oder 2, **gekennzeichnet durch** das

(1) Mischen eines oder mehrerer organischer $C_1$-$C_6$-Alkoxy-Silane der Formel (IV) mit Wasser,

$$R_9Si(OR_{10})_k(R_{11})_m \qquad (IV),$$

wobei

- $R_9$ für eine $C_1$-$C_{12}$-Alkylgruppe steht,
- $R_{10}$ für eine $C_1$-$C_6$-Alkylgruppe steht,
- $R_{11}$ für eine $C_1$-$C_6$-Alkylgruppe steht
- k für eine ganze Zahl von 1 bis 3 steht, und
- m für die ganze Zahl 3 - k steht.

**4.** Verfahren nach einem der Ansprüche 1 bis 3, **gekennzeichnet durch** das

(1) Mischen eines oder mehrerer organischer $C_1$-$C_6$-Alkoxy-Silane mit Wasser in einem Reaktionsgefäß oder

einem Reaktor, bevorzugt in einem Doppelwandreaktor, einem Reaktor mit externem Wärmeaustauscher, einem Rohrreaktor, einem Reaktor mit Dünnfilm-Verdampfer, einem Reaktor mit Fallfilmverdampfer und/oder einem Reaktor mit angeschlossenem Kondensator.

**5.** Verfahren nach einem der Ansprüche 1 bis 4, **gekennzeichnet durch** das

(1) Mischen eines oder mehrerer organischer $C_1$-$C_6$-Alkoxy-Silane mit 0,10 bis 0,80 Stoffmengenäquivalenten Wasser (S-W), bevorzugt von 0,15 bis 0,70, weiter bevorzugt von 0,20 bis 0,60 und ganz besonders bevorzugt von 0,25 bis 0,50 Stoffmengenäquivalenten Wasser,
wobei die Stoffmengenäquivalente Wasser sich berechnen nach der Formel

$$\text{S-W} = \frac{mol(Wasser)}{mol(Silane)\ x\ n(Alkoxy)}$$

mit

S-W = Stoffmengenäquivalent Wasser
mol(Wasser) = eingesetzte Molmenge Wasser
mol(Silane) = Gesamtmolmenge der in der Reaktion eingesetzten $C_1$-$C_6$-Alkoxy-Silane
n(Alkoxy) = Anzahl der $C_1$-$C_6$-Alkoxygruppen pro $C_1$-$C_6$-Alkoxy-Silan

**6.** Verfahren nach einem der Ansprüche 1 bis 5, **gekennzeichnet durch** die

(1) Reaktion eines oder mehrerer organischer $C_1$-$C_6$-Alkoxy-Silane mit Wasser bei einer Temperatur von 20 bis 65 °C, bevorzugt von 20 bis 60 °C, weiter bevorzugt von 20 bis 55 °C, noch weiter bevorzugt von 20 bis 50 °C und ganz besonders bevorzugt von 20 bis 45 °C.

**7.** Verfahren nach einem der Ansprüche 1 bis 6, **gekennzeichnet durch** die
(2) partielle oder vollständige Entfernung der durch die Reaktion in Schritt (1) freigesetzten $C_1$-$C_6$-Alkohole aus dem Reaktionsgemisch bei einer Temperatur von 20 bis 65 °C, bevorzugt von 20 bis 60 °C, weiter bevorzugt von 20 bis 55 °C, noch weiter bevorzugt von 20 bis 50 °C und ganz besonders bevorzugt von 20 bis 45 °C.

**8.** Verfahren nach einem der Ansprüche 1 bis 7, **gekennzeichnet durch** die
(2) partielle oder vollständige Entfernung der durch die Reaktion in Schritt (1) freigesetzten $C_1$-$C_6$-Alkohole aus dem Reaktionsgemisch mittels Destillation bei einem Druck von 0 bis 900 mbar, weiter bevorzugt von 0 bis 800 mbar, noch weiter bevorzugt von 200 bis 600 mbar und ganz besonders bevorzugt von 10 bis 300 mbar.

**9.** Verfahren nach einem der Ansprüche 1 bis 8, **gekennzeichnet durch** die
(2) partielle oder vollständige Entfernung der durch die Reaktion in Schritt (1) freigesetzten $C_1$-$C_6$-Alkohole aus dem Reaktionsgemisch mittels Destillation über einen Zeitraum von 90 bis 300 Minuten, bevorzugt von 120 bis 300 Minuten, weiter bevorzugt von 150 bis 300 Minuten und ganz besonders bevorzugt von 180 bis 300 Minuten.

**10.** Verfahren nach einem der Ansprüche 1 bis 9, **gekennzeichnet durch** die
(3) Zugabe eines oder mehrerer kosmetischer Inhaltsstoffe aus der Gruppe der Polymere, der oberflächenaktiven Verbindungen, der farbgebenden Verbindungen, der Fettkomponenten, der pH-Regulatoren, der Parfüme, der Konservierungsmittel, der Pflanzenextrakte und der Proteinhydrolysate.

**11.** Verfahren nach einem der Ansprüche 1 bis 10, **gekennzeichnet durch** die
(3) Zugabe eines oder mehrerer kosmetischer Inhaltsstoffe aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan.

**12.** Verfahren nach einem der Ansprüche 1 bis 11, **gekennzeichnet durch** die
(4) Abfüllung der Zubereitung in eine Flasche, eine Tube, einen Tiegel, eine Dose, ein Sachet, einen Aerosol-Druckbehälter, einen Non-Aerosol-Druckbehälter, einen Kanister oder einen Hobbock.

13. Verfahren nach einem der Ansprüche 1 bis 12, **dadurch gekennzeichnet, dass** der Gesamtgehalt an $C_1$-$C_6$-Alkoholen in der nach Schritt (4) erhaltenen Zubereitung - bezogen auf das Gesamtgewicht der Zubereitung - unterhalb von 9,0 Gew.-%, bevorzugt unterhalb von 8,0 Gew.-%, weiter bevorzugt unterhalb von 7,0 Gew.-%, noch weiter bevorzugt unterhalb von 6,0 Gew.-% und ganz besonders bevorzugt unterhalb von 5,0 Gew.-% liegt.

14. Verfahren nach einem der Ansprüche 1 bis 13, **dadurch gekennzeichnet, dass** der Wassergehalt in der nach Schritt (4) erhaltenen Zubereitung - bezogen auf das Gesamtgewicht der Zubereitung - unterhalb von 5,0 Gew.-%, bevorzugt unterhalb von 4,0 Gew.-%, weiter bevorzugt unterhalb von 3,0 Gew.-%, noch weiter bevorzugt unterhalb von 2,0 Gew.-% und ganz besonders bevorzugt unterhalb von 1,0 Gew.-% liegt.

15. Verfahren nach einem der Ansprüche 1 bis 14, umfassend die Schritte in der folgenden Reihenfolge:

(1) Reaktion eines oder mehrerer organischer $C_1$-$C_6$-Alkoxy-Silane mit Wasser,
(2) partielle oder vollständige Entfernung der durch die Reaktion in Schritt (1) freigesetzten $C_1$-$C_6$-Alkohole aus dem Reaktionsgemisch,
(3) gegebenenfalls Zugabe eines oder mehrerer kosmetischer Inhaltsstoffe, und
(4) Abfüllung der Zubereitung in eine Verpackungseinheit,

16. Verfahren nach einem der Ansprüche 1 bis 14, umfassend die Schritte in der folgenden Reihenfolge:

(1) Reaktion eines oder mehrerer organischer $C_1$-$C_6$-Alkoxy-Silane mit Wasser,
(3) gegebenenfalls Zugabe eines oder mehrerer kosmetischer Inhaltsstoffe,
(2) partielle oder vollständige Entfernung der durch die Reaktion in Schritt (1) freigesetzten $C_1$-$C_6$-Alkohole aus dem Reaktionsgemisch, und
(4) Abfüllung der Zubereitung in eine Verpackungseinheit.

17. Verfahren nach einem der Ansprüche 1 bis 16, **dadurch gekennzeichnet, dass** ein Mittel zur Färbung von keratinischem Material, zur Pflege von keratinischem Material oder zur Formveränderung von keratinischem Material hergestellt wird.

18. Mittel zur Behandlung von keratinischem Material, umfassend eine Zubereitung in einer Verpackungseinheit, die nach einem Verfahren hergestellt wurde, das in den Ansprüchen 1 bis 17 beschrieben ist.

19. Mehrkomponenten-Verpackungseinheit (Kit-of-parts) zum Färben von keratinischem Material, insbesondere menschlichen Haaren, welche getrennt voneinander konfektioniert

- eine erste Verpackungseinheit enthaltend eine kosmetische Zubereitung (A) und
- eine zweite Verpackungseinheit enthaltend eine kosmetische Zubereitung (B) umfasst, wobei
- die kosmetische Zubereitung (A) in der ersten Verpackungseinheit nach dem Verfahren hergestellt wurde, das in den Ansprüchen 1 bis 17 beschrieben ist, und
- die kosmetische Zubereitung (B) mindestens eine farbgebende Verbindung aus der Gruppe der Pigmente, der direktziehenden Farbstoffe und/oder der Oxidationsfarbstoffvorprodukte enthält.

## Claims

1. Process for the preparation of an agent for the treatment of keratinous material, in particular human hair, comprising the following steps:

(1) mixing one or more organic $C_1$-$C_6$-alkoxy silanes with water,
(2) partial or complete removal from the reaction mixture of the $C_1$-$C_6$ alcohols released by the reaction in step (1),
(3) where appropriate, addition of one or more cosmetic ingredients, and
(4) filling the preparation into a packaging unit,

**characterized in that** the total content of $C_1$-$C_6$ alcohols in the preparation obtained in step (4)

- based on the total weight of the preparation - is below 10.0% by weight.

**2.** Process according to claim 1, **characterized by** the

(1) mixing one or more organic $C_1$-$C_6$-alkoxy silanes of formula (I) and/or (II) with water,

$$R_1R_2N\text{-}L\text{-}Si(OR_3)a(R_4)_b \qquad (I)$$

where

- $R_1$ , $R_2$ independently represent a hydrogen atom or a $C_1$ -$C_6$ alkyl group,
- L is a linear or branched divalent $C_1$ -$C_{20}$ alkylene group,
- $R_3$, $R_4$ independently represent a $C_1$ -$C_6$ alkyl group,
- a, represents an integer from 1 to 3, and
- b is the integer 3 - a, and

$$(R_5O)_c(R_6)_dSi\text{-}(A)_e\text{-}[NR_7\text{-}(A')]_f\text{-}[O\text{-}(A'']_g\text{-}[NR_8\text{-}(A''')]_h\text{-}Si(R_6')_{d'}(OR_5')_{c'} \qquad (II),$$

where

- R5, R5', R5", R6, R6' and R6" independently represent a $C_1$-$C_6$ alkyl group,
- A, A', A", A‴ and A″″ independently represent a linear or branched divalent $C_1$ -$C_{20}$ alkylene group,
- $R_7$ and $R_8$ independently represent a hydrogen atom, a $C_1$ -$C_6$ alkyl group, a hydroxy-$C_1$ -$C_6$ alkyl group, a $C_2$ -$C_6$ alkenyl group, an amino-C -$C_{16}$ alkyl group or a grouping of formula (III), -

$$(A'''')\text{-}Si(R_6'')_d{}''(OR_5'')_c{}'' \qquad (III),$$

- c, stands for an integer from 1 to 3,
- d stands for the integer 3 - c,
- c' stands for an integer from 1 to 3,
- d' stands for the integer 3 - c',
- c" stands for an integer from 1 to 3,
- d" stands for the integer 3 - c",
- e stands for 0 or 1,
- f stands for 0 or 1,
- g stands for 0 or 1,
- h stands for 0 or 1,
- with the proviso that at least one of the residues from e, f, g and h is different from 0.

**3.** Process according to one of claims 1 or 2, **characterized by** the

(1) mixing one or more organic $C_1$-$C_6$-alkoxy silanes of formula (IV) with water,

$$R_9Si(OR_{10})_k(R_{11})m \qquad (IV),$$

where

- $R_9$ represents a $C_1$ -$C_{12}$ alkyl group,
- $R_{10}$ represents a $C_1$ -$C_6$ alkyl group,
- $R_{11}$ represents a $C_1$ -$C_6$ alkyl group
- k is an integer from 1 to 3, and
- m stands for the integer 3 - k.

**4.** Process according to any one of claims 1 to 3, **characterized by** the

(1) mixing one or more organic $C_1$-$C_6$-alkoxy silanes with water in a reaction vessel or reactor, preferably in a double-wall reactor, a reactor with external heat exchanger, a tubular reactor, a reactor with thin-film evaporator, a reactor with falling-film evaporator and/or a reactor with attached condenser.

**5.** Process according to any one of claims 1 to 4, **characterized by** the

(1) mixing one or more organic $C_1$-$C_6$-alkoxy silanes with from 0.10 to 0.80 molar equivalents of water (S-W), preferably from 0.15 to 0.70, more preferably from 0.20 to 0.60, and most preferably from 0.25 to 0.50 molar equivalents of water, where the mass equivalents of water are calculated according to the formula

$$\text{S-W} = \frac{mol(Wasser)}{mol(Silane)\ x\ n(Alkoxy)}$$

with

S-W = Mass equivalent water
mol(water) = molar quantity of water used
mol(silanes) = total molar amount of $C_1$-$C_6$-alkoxy silanes used in the reaction
n(alkoxy) = number of $C_1$-$C_6$-alkoxy groups per $C_1$-$C_6$-alkoxy silane.

**6.** Process according to any one of claims 1 to 5, **characterized by** the

(1) reaction of one or more organic $C_1$-$C_6$-alkoxy silanes with water at a temperature of from 20 to 65 °C, preferably from 20 to 60 °C, more preferably from 20 to 55 °C, still more preferably from 20 to 50 °C, and most preferably from 20 to 45 °C.

**7.** Process according to any one of claims 1 to 6, **characterized by** the
(2) partial or complete removal from the reaction mixture of the $C_1$ -$C_6$ alcohols liberated by the reaction in step (1) at a temperature of from 20 to 65 °C, preferably from 20 to 60 °C, more preferably from 20 to 55 °C, still more preferably from 20 to 50 °C, and most preferably from 20 to 45 °C.

**8.** Process according to any one of claims 1 to 7, **characterized by** the
(2) partial or complete removal of the $C_1$-$C_6$ alcohols released by the reaction in step (1) from the reaction mixture by distillation at a pressure of from 0 to 900 mbar, more preferably from 0 to 800 mbar, still more preferably from 200 to 600 mbar and most preferably from 10 to 300 mbar.

**9.** Process according to any one of claims 1 to 8, **characterized by** the
(2) partial or complete removal of the $C_1$ -$C_6$ alcohols released by the reaction in step (1) from the reaction mixture by distillation over a period of from 90 to 300 minutes, preferably from 120 to 300 minutes, more preferably from 150 to 300 minutes and most preferably from 180 to 300 minutes.

**10.** Process according to any one of claims 1 to 9, **characterized by** the
(3) addition of one or more cosmetic ingredients from the group of polymers, surface-active compounds, coloring compounds, fatty components, pH regulators, perfumes, preservatives, plant extracts and protein hydrolysates.

**11.** Process according to any one of claims 1 to 10, **characterized by** the
(3) Addition of one or more cosmetic ingredients selected from the group consisting of hexamethyldisiloxane, octamethyltrisiloxane, decamethyltetrasiloxane, hexamethylcyclotrisiloxane, octamethylcyclotetrasiloxane and/or decamethylcyclopentasiloxane.

**12.** Process according to any one of claims 1 to 11, **characterized by** the
(4) filling the preparation into a bottle, tube, jar, can, sachet, aerosol pressure container, non-aerosol pressure container, canister, or hobbock.

**13.** Process according to any one of claims 1 to 12, **characterized in that** the total content of $C_1$ - $C_6$ alcohols in the preparation obtained according to step (4) - based on the total weight of the preparation - is below 9.0% by weight, preferably below 8.0% by weight, more preferably below 7.0% by weight, still more preferably below 6.0% by weight and very particularly preferably below 5.0% by weight.

**14.** Process according to any one of claims 1 to 13, **characterized in that** the water content in the preparation obtained

after step (4) - based on the total weight of the preparation - is below 5.0% by weight, preferably below 4.0% by weight, more preferably below 3.0% by weight, even more preferably below 2.0% by weight and very particularly preferably below 1.0% by weight.

**15.** Process of any one of claims 1 to 14, comprising the steps in the following order:

(1) reaction of one or more organic $C_1$-$C_6$-alkoxy silanes with water,
(2) partial or complete removal from the reaction mixture of the $C_1$ -$C_6$ alcohols released by the reaction in step (1),
(3) where appropriate, addition of one or more cosmetic ingredients, and
(4) filling the preparation into a packaging unit,

**16.** Process of any one of claims 1 to 14, comprising the steps in the following order:

(1) Reaction of one or more organic $C_1$-$C_6$-alkoxy silanes with water,
(3) if necessary, addition of one or more cosmetic ingredients,
(2) partial or complete removal from the reaction mixture of the $C_1$ -$C_6$ alcohols released by the reaction in step (1), and
(4) Filling the preparation into a packaging unit.

**17.** Process according to any one of claims 1 to 16, **characterized in that** an agent for coloring keratinous material, for maintaining keratinous material or for changing the shape of keratinous material is prepared.

**18.** An agent for treating keratinous material comprising a preparation in a packaging unit prepared by a method described in claims 1 to 17.

**19.** Multi-component packaging unit (kit-of-parts) for dyeing keratinous material, in particular human hair, which are made up separately from each other.

- a first packaging unit containing a cosmetic preparation (A) and
- a second packaging unit containing a cosmetic preparation (B),
where
- the cosmetic preparation (A) in the first packaging unit has been prepared by the method described in claims 1 to 17, and
- the cosmetic formulation (B) comprises at least one colorant compound selected from the group consisting of pigments, direct dyes and/or oxidation dye precursors.

**Revendications**

**1.** Procédé de préparation d’une composition pour le traitement des matières kératiniques, en particulier des cheveux humains, comprenant les étapes suivantes :

(1) Mélange d’un ou de plusieurs $C_1$-$C_6$-alcoxy-silanes organiques avec de l’eau,
(2) élimination partielle ou totale du mélange réactionnel des alcools $C_1$ -$C_6$ libérés par la réaction de l’étape (1),
(3) l’addition éventuelle d’un ou de plusieurs ingrédients cosmétiques, et
(4) conditionnement de la préparation dans une unité d’emballage,

**caractérisé en ce que** la teneur totale en alcools $C_1$ -$C_6$ dans la préparation obtenue après l’étape (4) - par rapport au poids total de la préparation - est inférieure à 10,0 % en poids.

**2.** Procédé selon la revendication 1, **caractérisé par le fait que**

(1) mélanger un ou plusieurs $C_1$ -$C_6$-alcoxy-silanes organiques de formule (I) et/ou (II) avec de l’eau,

$$R_1R_2N\text{-}L\text{-}Si(OR_3)_a(R_4)_b \qquad (I)$$

où

- $R_1$ , $R_2$ représentent indépendamment un atome d'hydrogène ou un groupe alkyle en $C_1$ -$C_6$
- L représente un groupe alkylène divalent en $C_1$ -$C_{20}$ linéaire ou ramifié,
- $R_3$, $R_4$ représentent indépendamment l'un de l'autre un groupe alkyle en $C_1$ -$C_6$,
- a, représente un nombre entier de 1 à 3, et
- b représente le nombre entier 3 - a, et

$$(R_5O)_c(R_6)_dSi\text{-}(A)_e\text{-}[NR_7\text{ -}(A')]_f\text{ -}[O\text{-}(A'')]_g\text{-}[NR_8\text{ -}(A''')]_h\text{ -}Si(R_6')_{d'}(OR_5')_{c'} \qquad (II),$$

où

- R5, R5', R5", R6, R6' et R6" représentent indépendamment un groupe alkyle en Ci-Ce,
- A, A', A" A‴ et A'''' représentent indépendamment un groupe alkylène divalent en $C_1$-$C_{20}$ linéaire ou ramifié,
- $R_7$ et $R_8$ représentent indépendamment l'un de l'autre un atome d'hydrogène, un groupe alkyle en Ci-Ce , un groupe hydroxy-$C_1$-$C_6$-alkyle, un groupe alcényle en $C_2$-$C_6$ , un groupe amino-$C_1$-$C_6$-alkyle ou un groupement de formule (III), -

(A"")-Si(R6")d"(OR5")c" (III),

- c, représente un nombre entier de 1 à 3,
- d représente le nombre entier 3 - c,
- c' représente un nombre entier de 1 à 3,
- d' représente le nombre entier 3 - c',
- c" représente un nombre entier de 1 à 3,
- d" représente le nombre entier 3 - c",
- e est égal à 0 ou 1,
- f est égal à 0 ou 1,
- g est égal à 0 ou 1,
- h est égal à 0 ou 1,
- à condition qu'au moins l'un des radicaux parmi e, f, g et h soit différent de 0.

3. Procédé selon l'une des revendications 1 ou 2, **caractérisé par le fait que**

(1) mélanger un ou plusieurs $C_1$-$C_6$-alcoxy-silanes organiques de formule (IV) avec de l'eau,

$$R_9Si(OR_{10})_k(R_{11})_m \qquad (IV),$$

où

- $R_9$ représente un groupe alkyle en $C_1$ -$C_{12}$
- $R_{10}$ représente un groupe alkyle en $C_1$ -$C_6$
- $R_{11}$ représente un groupe alkyle en $C_1$ -$C_6$
- k représente un nombre entier de 1 à 3, et
- m représente le nombre entier 3 - k.

4. Procédé selon l'une quelconque des revendications 1 à 3, **caractérisé par le fait que**

(1) mélanger un ou plusieurs $C_1$-$C_6$-alcoxy-silanes organiques avec de l'eau dans un récipient de réaction ou un réacteur, de préférence dans un réacteur à double paroi, un réacteur avec échangeur de chaleur externe, un réacteur tubulaire, un réacteur avec évaporateur à film mince, un réacteur avec évaporateur à film tombant et/ou un réacteur avec condenseur raccordé.

5. Procédé selon l'une quelconque des revendications 1 à 4, **caractérisé par le fait que**

(1) mélanger un ou plusieurs $C_1$ -$C_6$ -alcoxy-silanes organiques avec 0,10 à 0,80 équivalent de quantité de matière d'eau (S-W), de préférence de 0,15 à 0,70, plus préférablement de 0,20 à 0,60 et tout particulièrement

de 0,25 à 0,50 équivalent de quantité de matière d'eau, où les équivalents en quantité de matière d'eau se calculent selon la formule

$$S\text{-}W = \frac{mol(Wasser)}{mol(Silane)\ x\ n(Alkoxy)}$$

avec

S-W = équivalent de quantité de matière eau
mol(eau) = quantité de moles d'eau utilisée
mol(silanes) = quantité molaire totale des $C_1$ -$C_6$ -alcoxy-silanes utilisés dans la réaction
n(alcoxy) = nombre de groupes $C_1$ -$C_6$ -alcoxy par $C_1$ -$C_6$ -alcoxy-silane.

**6.** Procédé selon l'une quelconque des revendications 1 à 5, **caractérisé en ce que**

(1) réaction d'un ou plusieurs alcoxy silanes organiques Ci-Ce avec de l'eau à une température de 20 à 65°C, de préférence de 20 à 60°C, de préférence encore de 20 à 55°C, de préférence encore de 20 à 50°C et de préférence encore de 20 à 45°C.

**7.** Procédé selon l'une quelconque des revendications 1 à 6, **caractérisé en ce que** (2) élimination partielle ou totale du mélange réactionnel des alcools Ci-Ce libérés par la réaction de l'étape (1) à une température de 20 à 65°C, de préférence de 20 à 60°C, plus préférablement de 20 à 55°C, encore plus préférablement de 20 à 50°C et tout particulièrement de 20 à 45°C.

**8.** Procédé selon l'une quelconque des revendications 1 à 7, **caractérisé en ce que** (2) élimination partielle ou totale des alcools Ci-Ce libérés par la réaction dans l'étape (1) du mélange réactionnel par distillation à une pression de 0 à 900 mbar, de préférence encore de 0 à 800 mbar, de préférence encore de 200 à 600 mbar et de manière tout à fait préférée de 10 à 300 mbar.

**9.** Procédé selon l'une quelconque des revendications 1 à 8, **caractérisé par le fait que** (2) élimination partielle ou totale des alcools Ci-Ce libérés par la réaction dans l'étape (1) du mélange réactionnel par distillation pendant une période de 90 à 300 minutes, de préférence de 120 à 300 minutes, de manière encore plus préférée de 150 à 300 minutes et de manière tout particulièrement préférée de 180 à 300 minutes.

**10.** Procédé selon l'une quelconque des revendications 1 à 9, **caractérisé en ce que** (3) ajout d'un ou plusieurs ingrédients cosmétiques choisis dans le groupe des polymères, des composés tensioactifs, des composés colorants, des composants gras, des régulateurs de pH, des parfums, des conservateurs, des extraits de plantes et des hydrolysats de protéines.

**11.** Procédé selon l'une quelconque des revendications 1 à 10, **caractérisé en ce que** (3) addition d'un ou plusieurs ingrédients cosmétiques choisis dans le groupe constitué par l'hexaméthyldisiloxane, l'octaméthyltrisiloxane, le décaméthyltétrasiloxane, l'hexaméthylcyclotrisiloxane, l'octaméthylcyclotétrasiloxane et/ou le décaméthyl-cyclopentasiloxane.

**12.** Procédé selon l'une quelconque des revendications 1 à 11, **caractérisé par le fait que** (4) conditionnement de la préparation dans une bouteille, un tube, un pot, une boîte, un sachet, un récipient sous pression pour aérosol, un récipient sous pression sans aérosol, un bidon ou un hobbock.

**13.** Procédé selon l'une des revendications 1 à 12, **caractérisé en ce que** la teneur totale en alcools $C_1$-$C_6$ dans la préparation obtenue après l'étape (4) - par rapport au poids total de la préparation - est inférieure à 9,0 % en poids, de préférence inférieure à 8,0 % en poids, plus préférablement inférieure à 7,0 % en poids, encore plus préférablement inférieure à 6,0 % en poids et tout particulièrement inférieure à 5,0 % en poids.

**14.** Procédé selon l'une des revendications 1 à 13, **caractérisé en ce que** la teneur en eau dans la préparation obtenue après l'étape (4) - par rapport au poids total de la préparation - est inférieure à 5,0 % en poids, de préférence inférieure à 4,0 % en poids, plus préférentiellement inférieure à 3,0 % en poids, encore plus préférentiellement

inférieure à 2,0 % en poids et tout particulièrement inférieure à 1,0 % en poids.

**15.** Procédé selon l'une quelconque des revendications 1 à 14, comprenant les étapes dans l'ordre suivant :

(1) réaction d'un ou de plusieurs $C_1$ -$C_6$ -alcoxy-silanes organiques avec l'eau,
(2) élimination partielle ou totale du mélange réactionnel des alcools $C_1$ -$C_6$ libérés par la réaction de l'étape (1),
(3) l'addition éventuelle d'un ou de plusieurs ingrédients cosmétiques, et
(4) conditionnement de la préparation dans une unité d'emballage,

**16.** Procédé selon l'une quelconque des revendications 1 à 14, comprenant les étapes dans l'ordre suivant :

(1) réaction d'un ou de plusieurs $C_1$ -$C_6$ -alcoxy-silanes organiques avec l'eau,
(3) l'addition éventuelle d'un ou plusieurs ingrédients cosmétiques,
(2) élimination partielle ou totale du mélange réactionnel des alcools $C_1$ -$C_6$ libérés par la réaction de l'étape (1), et
(4) conditionnement de la préparation dans une unité d'emballage.

**17.** Procédé selon l'une des revendications 1 à 16, **caractérisé en ce que** l'on prépare un agent de coloration de la matière kératinique, de soin de la matière kératinique ou de modification de la forme de la matière kératinique.

**18.** Composition pour le traitement de la matière kératinique, comprenant une préparation dans une unité d'emballage, préparée selon un procédé décrit dans les revendications 1 à 17.

**19.** Unité d'emballage à plusieurs composants (kit of parts) pour la coloration de matières kératiniques, en particulier de cheveux humains, qui sont confectionnés séparément les uns des autres

- une première unité d'emballage contenant une préparation cosmétique (A) et
- une deuxième unité d'emballage contenant une préparation cosmétique (B),
où
- la préparation cosmétique (A) dans la première unité d'emballage a été fabriquée selon le procédé décrit dans les revendications 1 à 17, et
- la préparation cosmétique (B) contient au moins un composé colorant du groupe des pigments, des colorants à action directe et/ou des précurseurs de colorants d'oxydation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2168633 B1
- WO 2013068979 A2